# EUROPEAN PATENT SPECIFICATION

(11) **EP 1 651 684 B1**
(45) Date of publication and mention of the grant of the patent: **07.11.2007**
(21) Application number: 04757418.1
(22) Date of filing: 04.08.2004
(51) Int. Cl.: C08F 8/30, C08F 10/10, C08F 20/00

(54) **ETHERIFIED CARBAMATE FUNCTIONAL COPOLYMERS OF ISOBUTYLENE TYPE MONOMERS, AND THEIR USE IN CURABLE COMPOSITIONS**
COPOLYMERE VON ISOBUTYLENARTIGEN MONOMEREN MIT VERETHERTEN CARBAMINSÄUREESTERGRUPPEN UND DEREN VERWENDUNG IN VERNETZBAREN ZUSAMMENSETZUNGEN
COPOLYMERES FONCTIONNELS DE CARBAMATE ETHERIFIE DE MONOMERES DE TYPE ISOBUTYLENE ET LEUR UTILISATION DANS DES COMPOSITIONS DURCISSABLES

(30) Priority: 06.08.2003 US 635796
(43) Date of publication of application: 03.05.2006
(73) Proprietor: PPG Industries Ohio, Inc., Cleveland, OH 44111 (US)
(72) Inventor: O'DWYER, James B., Valencia, PA 16059 (US); COCA, Simion, Pittsburgh, PA 15223 (US); BARANCYK, Steven V., Wexford, PA 15090 (US)
(74) Representative: Polypatent
(86) International application number: PCT/US2004/025088
(87) International publication number: WO 2005/016979

(56) References cited:
- WO-A-00/12566
- WO-A-00/31195
- WO-A-01/14431
- WO-A-01/23484
- WO-A-94/10212
- WO-A-97/47664
- WO-A-03/070783
- WO-A-03/070825
- US-A- 3 284 415

## Description

### Field of the Invention

The present invention relates to reaction products having etherified carbamate functionality, and to curable compositions containing them.

### Background of the Invention

Functional polymers used in coating compositions are typically random copolymers that include functional group-containing acrylic and/or methacrylic monomers. Such a functional copolymer will contain a mixture of polymer molecules having varying individual functional equivalent weights and polymer chain structures. In such a copolymer, the functional groups are located randomly along the polymer chain. Moreover, the number of functional groups is not divided equally among the polymer molecules, such that some polymer molecules may actually be free of functionality.

In a thermosetting composition, the formation of a three-dimensional crosslinked network is dependent on the functional equivalent weight as well as the architecture of the individual polymer molecules that comprise it. Polymer molecules having little or no reactive functionality (or having functional groups that are unlikely to participate in crosslinking reactions due to their locations along the polymer chain) will contribute little or nothing to the formation of the three-dimensional crosslinked network, resulting in decreased crosslink density and less than optimum physical properties of the finally formed thermoset coating.

Many patents express the potential for using isobutylene-containing polymers in coating compositions. For example, U.S. Patent No. 6,114,489 to Vicari et al. discloses a coating composition that includes a functional acrylic resin binder; a co-reactant capable of reacting with the functionality of the acrylic binder; a degasser; and a hyperbranched polyester flow and leveling agent. Isobutylene is suggested as a potential co-monomer for use in the acrylic binder as part of a long list of monomers. U.S. Patent No. 5,552,487 to Clark et al. discloses powder coating compositions that include a copolymer having a reactive functionality and a suitable crosslinking agent capable of reaction with the reactive functionality of the copolymer. The copolymer is a made by copolymerizing functional monomers with other monomers, isobutylene being one among many listed as potential co-monomers. Although only two are referenced herein, of the many patents that express the possibility of using isobutylene-type co-monomers, none actually shows or discloses a working example of such a copolymer.

The fact that no examples of isobutylene-type monomer-containing copolymers in coating compositions can be found is most likely due to the generally non-reactive nature of isobutylene with acrylic and methacrylic monomers. Reactivity ratios for monomers can be calculated using the Alfrey - Price Q-e values (Robert Z. Greenley, Polymer Handbook, Fourth Edition, Brandrup, Immergut and Gulke, editors, Wiley & Sons, New York, NY, pp. 309-319 (1999)). The calculations may be carried out using the formulas I and II:

I r₁=(Q₁/Q₂)exp{-e₁(e₁-e₂)}

II r₂=(Q₂/Q₁)exp{-e₂(e₂-e₁)}

where r₁ and r₂ are the respective reactivity ratios of monomers 1 and 2,and Q₁ and Q₂ and e₁ and e₂ are the respective reactivity and polarity values for the respective monomers (Odian, Principals of Polymerization, 3rd Ed., Wiley-lnterscience, New York, NY, Chapter 6, pp. 452-467 and 489-491 (1991)). Table 1 shows the calculated reactivity ratios of selected monomers with isobutylene:

**Table 1**

| Monomer | r₁ (isobutylene) | r₂ |
|---|---|---|
| Methyl acrylate | 0.10 | 13.67 |
| Glycidyl methacrylate | 0.08 | 34.17 |
| Methacrylic acid | 0.09 | 39.71 |

As one skilled in the art of polymer chemistry can appreciate, when r₁ is near zero and r₂ has a value of 10 or more, monomer 2 is reactive toward both monomers and monomer 1 is reactive toward neither monomer. In other words, it is extremely difficult to prepare copolymers having significant amounts of both monomers. It is not surprising then that no examples can be found of coating compositions that include isobutylene-type monomer-containing copolymers, because the monomers do not tend to copolymerize.

In some cases, it is observed that monomers that do not readily homopolymerize are able to undergo rapid copolymerization reactions with each other. The most typical situation occurs when a strong electron donating monomer is mixed with a strong electron accepting monomer from which a regular alternating copolymer results after free radical initiation. Maleic anhydride is a widely used example of a strong electron accepting monomer. Styrene and vinyl ethers are typical examples of electron donating monomers. Systems, such as maleic anhydride - styrene, are known to form charge transfer complexes, which tend to place the monomers in alternating sequence prior to initiation. The application of the free radical initiator "ties" the ordered monomers together to form an alternating copolymer (Cowie, Alternating Copolymers, Plenum, New York (1985)).

U.S. Patent Nos. 2,378,629 to Hanford and 4.151,336 to Sackman et al. disclose that even when a moderately electron donating monomer, such as diisobutylene, is copolymerized with a strong electron acceptor monomer, such as maleic anhydride, an alternating copolymer results.

When a moderately electron donating monomer, such as isobutylene, is copolymerized with a moderately electron accepting monomer, such as an acrylic ester, poor incorporation of the electron donating monomer results. For example, free radical copolymerization of isobutylene (IB) and acrylic monomers has resulted in copolymers that contain at no more than 20-30% of IB and have low molecular weights because of the degradative chain transfer of IB. Examples of such copolymerizations of IB are disclosed by U.S. Patent Nos. 2,411,599 to Sparks et al. and 2,531,196 to Brubaker et al.

Conjugated monomers, such as acrylic esters and acrylonitrile, have been shown to react with monomers such as propylene, isobutylene, and styrene, in the presence of Lewis acids, such as alkylaluminum halides, to give 1:1 alternating copolymers. The alternating copolymers were obtained when the concentration ratio of the Lewis acids to the acrylic esters was 0.9 and the concentration of IB was greater than the concentration of the acrylic esters (Hirooka et al, J. Polym. Sci. Polym. Chem., 11, 1281 (1973)). The metal halides vary the reactivity of the monomers by complexing with them. The electron donor monomer - electron acceptor monomer - metal halide complex leads to alternating copolymers (Mashita et al. Polymer, Vol. 36, No. 15, pp. 2973-2982. (1995)).

Copolymers of IB and methyl acrylate (MA) have also been obtained by using ethyl aluminum sesquichloride and 2-methyl pentanoyl peroxide as an initiating system. The resulting copolymer had an alternating structure, with either low (Kuntz et al, J. Polym. Sci. Polym. Chem., 16, 1747 (1978)) or high isotacticity in the presence of EtAlCl₂ (10 molar % relative to MA). (Florjanczyk et al, Makromol. Chem., 183, 1081 (1982)).

Another method for making IB copolymers with acrylic esters involved alkyl boron halide, which was found to be much more active than alkyl aluminum halides in forming alternating copolymers. The resulting copolymer was an elastomer of high tensile strength and high thermal decomposition temperature with good oil resistance, especially at elevated temperatures (Mashita et al, Polymer, 36, 2983 (1995)).

U.S. Patent No. 5,807,937 to Matyjaszewski et al. discloses a method of making alternating copolymers of isobutylene and methyl acrylate using an atom transfer radical polymerization (ATRP) process. The method requires the use of a suitable ATRP initiator, such as 1-phenylethyl bromide, and a suitable transition metal salt, such as CuBr with a ligand, such as 2,2'-bipyridyl to perform the complex redox initiation and propagation steps of the polymerization process.

WO00/12566 discloses a thermoset composition containing a carbamate-functional polymer. The composition comprises a polymer comprising a monomer "M", free of carbamate residues and a monomer "G" containing carbamate residues. The monomer "M" may be isobutene (see page 10, line 8). In one embodiment, the monomer "G" and "M"may form an alternating copolymer (see page 8, lines 5-11).
Moreover, the composition comprises a crosslinking agent.
The crosslinking agent may be selected from aminoplasts containing methylol and/or methylol ether groups.

Copolymers containing relatively high amounts (≥ 30 mol %) of IB and acrylic esters have only been attained by free radical polymerization when Lewis acids or ATRP initiation systems have been employed. The polymer that results from such processes requires expensive and time consuming clean up to remove the transition metal salt and/or Lewis acid residues in order to make the polymer commercially useful.

Copolymer compositions that contain Lewis acids and/or transition metals intermingled with the copolymer can have a number of drawbacks when used commercially in coating compositions. First, some Lewis acids and transition metals are toxic and have adverse environmental effects if they are leached from the copolymer and enter the environment. Second, in coating applications the Lewis acids and transition metals may lead to poor color stability when the coating is exposed to UV light or simply cause the coating to discolor through other reactions or interactions. Further, the Lewis acids and transition metals may react with other ingredients in a coating formulation resulting in undesired properties, such as a shortened shelf-life for a given coating formulation.

Coating compositions used in the original automotive equipment market are being called to more and more stringent performance requirements; automotive manufacturers have very strict performance requirements of the coatings that are used. Coating systems are expected to provide lasting weatherability, durability, resistance to acid etching and water spotting, and mar resistance, while maintaining outstanding appearance properties.

Some coating compositions cured via acid-epoxy cure mechanisms, while providing excellent acid etch resistance, offer only marginal mar resistance. Conventional coating compositions cured with aminoplast crosslinking agents have been known for superior durability, but it has only been recently that aminoplast-cured coatings providing acid etch resistance have become available. Moreover, aminoplast-cured systems typically suffer from high photo-oxidation rates due to the breakdown of the aminotriazine ring inherently found in most aminoplast resins. Such degradation is due to prolonged exposure to ultraviolet light.

It would be desirable to provide crosslinking agents and curable compositions suitable for use as film-forming compositions in the automotive and industrial markets that overcome the drawbacks of the prior art, providing both appearance and performance properties now considered essential in automotive applications.

### Summary of the Invention

The present invention provides a reaction product of reactants comprising:
a) at least one copolymer comprising at least 30 mol % of residues having the following alternating structural units:

   -[DM-AM]-

   wherein DM represents a residue from a donor monomer, AM represents a residue from an acceptor monomer, at least 15 mol % of the copolymer comprising a donor monomer having the following structure (I): 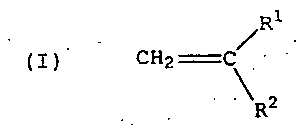 wherein R¹ is linear or branched C₁ to C₆ alkyl, R² is selected from the group consisting of linear, cyclic or branched C₁ to C₂₀ alkyl, alkenyl, C₆ to C₂₀ aryl, alkaryl and aralkyl, at least 15 mol % of the copolymer comprising an acrylic monomer as an acceptor monomer; the copolymer containing pendant carbamate groups or groups that can be converted to carbamate groups;
b) at least one aldehyde; and
c) at least one monohydric alcohol;
   wherein when the copolymer (a) contains groups that can be converted to carbamate groups, the reactants further comprise:
d) at least one material that will convert said groups into carbamate groups.

Also provided is a copolymer comprising at least 30 mol % of residues having the following alternating structural units:

-[DM-AM]-

wherein DM represents a residue from a donor monomer, AM represents a residue from an acceptor monomer, at least 15 mol % of the copolymer comprising a donor monomer having the following structure (I): 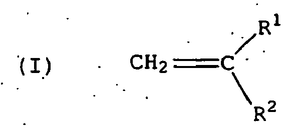 wherein R¹ and R² are as defined above, at least 15 mol % of the copolymer comprising an acrylic monomer as an acceptor monomer; the copolymer containing pendant groups of the structure:

-OC(O)N(R")CH₂OR'

where R' is alkyl containing one to eight carbon atoms and R" is selected from H,CH₂OR', linear, cyclic or branched C₁ to C₂₀ alkyl, alkenyl, C₆ to C₂₀ aryl, alkaryl and aralkyl. The reaction product and copolymer are suitable for use in a variety of curable compositions, which are also provided.

### Detailed Description of the Preferred Embodiments

Other than in the operating examples, or where otherwise indicated, all numbers expressing quantities of ingredients, reaction conditions and so forth used in the specification and claims are to be understood as being modified in all instances by the term "about." Accordingly, unless indicated to the contrary, the numerical parameters set forth in the following specification and attached claims are approximations that may vary depending upon the desired properties to be obtained by the present invention: At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical values, however, inherently contain certain errors necessarily resulting from the standard deviation found in their respective testing measurements.

The reaction product of the present invention is typically prepared by reacting together:
a) at least one copolymer comprising at least 30 mol % of residues having the following alternating structural units:

   -[DM-AM]-

   wherein DM represents a residue from a donor monomer, AM represents a residue from an acceptor monomer, at least 15 mol % of the copolymer comprising a donor monomer having the following structure (1): 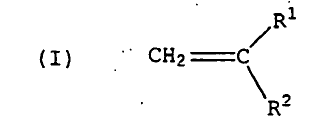 wherein R¹ is linear or branched C₁ to C₆ alkyl, R² is selected from the group consisting of linear, cyclic or branched C₁ to C₂₀ alkyl, alkenyl, C₆ to C₂₀ aryl, alkaryl and aralkyl, at least 15 mol % of the copolymer comprising an acrylic monomer as an acceptor monomer; the copolymer containing pendant carbamate groups or groups that can be converted to carbamate groups;.
b) at least one aldehyde; and
c) at least one monohydric alcohol;
   wherein when the copolymer (a) contains groups that can be converted to carbamate groups, the reactants further comprise:
d) at least one material that will convert said groups into carbamate groups.

The terms "donor monomer" and "acceptor monomer" are used throughout this application. With regard to the present invention, the term "donor monomer" refers to monomers that have a polymerizable, ethylenically unsaturated group that has relatively high electron density in the ethylenic double bond, and the term "acceptor monomer" refers to monomers that have a polymerizable, ethylenically unsaturated group that has relatively low electron density in the ethylenic double bond. This concept has been quantified to an extent by the Alfrey-Price Q-e scheme (Robert Z. Greenley, Polymer Handbook, Fourth Edition, Brandrup, lmmergut and Gulke, editors, Wiley & Sons, New York, NY, pp. 309-319 (1999)). All e values recited herein are those appearing in the Polymer Handbook unless otherwise indicated.

In the Q-e scheme, Q reflects the reactivity of a monomer and e represents the polarity of a monomer, which indicates the electron density of a given monomer's polymerizable, ethylenically unsaturated group. A positive value for e indicates that a monomer has a relatively low electron density and is an acceptor monomer, as is the case for maleic anhydride, which has an e value of 3.69. A low or negative value for e indicates that a monomer has a relatively high electron density and is a donor monomer, as is the case for vinyl ethyl ether, which has an e value of -1.80.

As referred to herein, a strong acceptor monomer is meant to include those monomers with an e value greater than 2.0. The term "mild acceptor monomer" is meant to include those monomers with an e value greater than 0.5 up to and including those monomers with an e value of 2.0. Conversely, the term "strong donor monomer" is meant to include those monomers with an e value of less than -1.5, and the term "mild donor monomer" is meant to include those monomers with an e value of less than 0.5 to those with an e value of -1.5.

The copolymer used to prepare the reaction product of the present invention comprises at least 30 mol %, in many cases at least 40 mol %, typically at least 50 mol %, in some cases at least 60 mol %, and in other cases at least 75 mol % of residues in the copolymer derived from alternating sequences of donor monomer - acceptor monomer pairs having the alternating monomer residue units of structure:

-[DM-AM]-

where DM represents a residue from a donor monomer and AM represents a residue from an acceptor monomer. The copolymer may be a 100% alternating copolymer of DM and AM. More particularly, at least 15 mol % of the copolymer comprises a donor monomer, which is an isobutylene-type monomer, having the following structure (I): 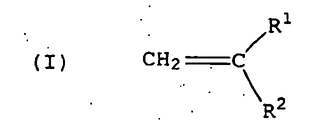 where R¹ and R² are as defined above. In a particular embodiment, at least 15 mol % of the copolymer includes at least one acrylic monomer as an acceptor monomer. The group R¹ is typically selected from at least one of methyl, ethyl, n-propyl, isopropyl, n-butyl, isobutyl, and cyclohexyl. The group R² may include one or more functional groups selected from hydroxy, epoxy, carboxylic acid, ether, carbamate, and amide. The group R² most often includes hydroxyl groups.

The copolymer incorporates a substantial portion of alternating residues of a mild donor monomer as described by structure I and a mild acceptor monomer, which is often an acrylic monomer. A non-limiting list of published e values for monomers that may be included as monomers described by structure I and acrylic monomers of the present invention are shown in Table 2.

**Table 2**

| Alfrey-Price e values for Selected Monomers | |
|---|---|
| Monomer | e value |
| Monomers of structure 1 | |
| Isobutylene | -1.20¹ |
| Diisobutylene | 0.49² |

| Acrylic Monomers | |
|---|---|
| Acrylic Acid | 0.88¹ |
| Acrylamide | 0.54¹ |
| Acrylonitrile | 1.23¹ |
| Methyl Acrylate | 0.64¹ |
| Ethyl Acrylate | 0.55¹ |
| Butyl Acrylate | 0.85¹ |
| Benzyl acrylate | 1.13¹ |
| Glycidyl acrylate | 1.28¹ |

| | |
|---|---|
| ¹Polymer Handbook, Fourth Edition (1999) ²Rzaev et al., Eur. Polym. J., Vol. 24, No. 7, pp. 981-985 (1998) | |

Typically, the copolymer used as reactant (a) in the preparation of the reaction product of the present invention is substantially free of maleate monomer residues and fumarate monomer residues, which usually have e values greater than 2.0. These types of multifunctional monomers provide too many functional groups to the copolymer. This can create problems, for example, in coatings where a thermosetting composition may have a short shelf-life due to the overly functional nature of the copolymer.

Further, the copolymer (a) is substantially free of transition metals and Lewis acids, which have been used in the prior art to make alternating copolymers of mild donor monomers and mild acceptor monomers. Transition metal or Lewis acid adjuncts are not utilized in preparing the copolymer (a), therefore, they do not need to be removed after polymerization and the resulting reaction products will not suffer the drawbacks inherent in those that contain transition metals or Lewis acids.

Any suitable donor monomer may be used in the preparation of the copolymer (a). Suitable donor monomers that may be used include strong donor monomers and mild donor monomers. Mild donor monomers are particularly useful for preparing alternating copolymers. The copolymers will include a mild donor monomer described by structure I, such as isobutylene and diisobutylene, dipentene, and isoprenol, and may additionally include other suitable mild donor monomers. The mild donor monomer of structure I is present in the copolymer (a) at a level of at least 15 mol %, in some cases at least 25 mol %, typically at least 30 mol % and in some cases at least 35 mol %. The mild donor monomer of structure I is present in the copolymer (a) at a level of up to 50 mol %, in some cases up to 47.5 mol %, typically up to 45 mol %, and, in some cases, up to 40 mol %. Residues from the mild donor monomer of structure I may be present in the copolymer (a) in any range of values inclusive of those stated above.

Suitable other donor monomers that may be used in the preparation of the copolymer include, but are not limited to, ethylene, butene, styrene, substituted styrenes, methyl styrene, substituted methyl styrenes, vinyl ethers, vinyl esters, vinyl pyridines, divinyl benzene, vinyl naphthalene, and divinyl naphthalene. Vinyl esters include vinyl esters of carboxylic acids, which include, but are not limited to, vinyl acetate, vinyl butyrate, vinyl 3,4-dimethoxybenzoate, and vinyl benzoate. The use of other donor monomers is optional. When other donor monomers are present, they are present at a level of at least 0.01 mol % of the copolymer composition, often at least 0.1 mol %, typically at least 1 mol %, and, in some cases, at least 2 mol %. The other donor monomers may be present at up to 25 mol %, in some cases up to 20 mol %, typically up to 10 mol %, and, in some cases, up to 5 mol %. Residues from the other donor monomers may be present in the copolymer (a) in any range of values inclusive of those stated above.

The copolymer (a) includes acceptor monomers as part of the alternating donor monomer - acceptor monomer units along the copolymer chain. It is to be understood that acceptor monomers as used in the preparation of the copolymer are not to be construed as Lewis acids, the use of which as catalysts is undesirable in the present invention as discussed above. Any suitable acceptor monomer may be used. Suitable acceptor monomers include strong acceptor monomers and mild acceptor monomers. A non-limiting class of suitable acceptor monomers are those described by the structure (II): 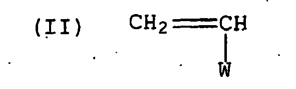 where W is selected from the group consisting of linear or branched C₁ to C₂₀ alkyl and alkylol, -CN, -X, and -C(=O)-Y, wherein Y is selected from the group consisting of -NR³₂, -O-R⁵-O-C(=O)-NR³₂, and -OR⁴. Each R³ may be the same or different and is selected from the group consisting of H, linear or branched C₁ to C₂₀ alkyl, and linear or branched C₁ to C₂₀ alkylol R⁴ is selected from the group consisting of H, poly(ethylene oxide), poly(propylene oxide), linear or branched C₁ to C₂₀ alkyl, including carbamoyl alkyl, alkylol, C₆ to C₂₀ aryl and aralkyl, linear or branched C₁ to C₂₀ fluoroalkyl, fluoroaryl and fluoroaralkyl, and a polysiloxane radical. R⁵ is a divalent linear or branched C₁ to C₂₀ alkyl linking group, and X is a halide. W is most often linear or branched C₁ to C₂₀ alkyl or alkylol.

A class of mild acceptor monomers that may be included in the present copolymer composition are acrylic acceptor monomers. Suitable acrylic acceptor monomers include those described by structure (III): 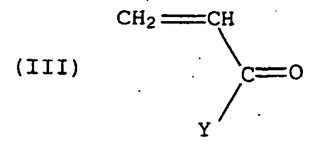 where Y is as defined above. Y is most often -OR⁴, and R⁴ is typically linear or branched C₁ to C₂₀ alkyl or alkylol.

Examples of suitable acceptor monomers include, but are not limited to, hydroxyethyl acrylate, hydroxypropyl acrylate, 4-hydroxybutyl acrylate, which is preferred, 2-carbamoyloxyethyl acrylate, 2-carbamoyloxypropyl acrylate, acrylic acid, methyl acrylate, ethyl acrylate, butyl acrylate, isobutyl acrylate, isobornyl acrylate, dimethylaminoethyl acrylate, acrylamide, perfluoro methyl ethyl acrylate, perfluoro ethyl ethyl acrylate, perfluoro butyl ethyl acrylate, trifluoromethyl benzyl acrylate, perfluoro alkyl ethyl, acryloxyalkyl terminated polydimethylsiloxane, acryloxyalkyl tris(trimethylsiloxy silane), and acryloxyalkyl trimethylsiloxy terminated polyethylene oxide, chlorotrifluoro ethylene, glycidyl acrylate, 2-ethylhexyl acrylate, and n-butoxy methyl acrylamide.

The acrylic acceptor monomers of structure III may be present in the copolymer (a) at a level of at least 15 mol %, in some cases at least 25 mol %, typically at least 30 mol %, and, in some cases, at least 35 mol %. The acrylic acceptor monomers of structure III may be present in the copolymer (a) at a level of up to 50 mol %, in some cases up to 47.5 mol %, typically up to 45 mol %, and, in some cases, up to 40 mol %. The level of the acrylic acceptor monomers of structure III used is determined by the properties that are to be incorporated into the copolymer composition. Residues from the acrylic acceptor monomers of structure III may be present in the copolymer (a) in any range of values inclusive of those stated above.

Suitable other mild acceptor monomers that may be used in the copolymer (a) include, but are not limited to, acrylonitrile, methacrylonitrile, vinyl halides, crotonic acid, vinyl alkyl sulfonates, and acrolein. Vinyl halides include, but are not limited to, vinyl chloride and vinylidene fluoride. The use of other mild acceptor monomers is optional, when other mild acceptor monomers are present, they are present at a level of at least 0.01 mol % of the copolymer (a), often at least 0.1 mol %, typically at least 1 mol %, and, in some cases, at least 2 mol %. The other acceptor monomers may be present at up to 35 mol %, in some cases up to 25 mol %, typically up to 15 mol %, and, in some cases, up to 10 mol %. The level of other acceptor monomers used is determined by the properties that are to be incorporated into the copolymer composition. Residues from the other acceptor monomers may be present in the copolymer (a) in any range of values inclusive of those stated above.

The copolymer (a) has a molecular weight of at least 250, in many cases at least 500, typically at least 1,000, and, in some cases, at least 2,000. The present copolymer may have a molecular weight of up to 1,000,000, in many cases up to 500,000, typically up to 100,000, and, in some cases, up to 50,000. Certain applications will require that the molecular weight of the copolymer (a) not exceed 30,000, in some cases not exceed 25,000, in other cases not exceed 20,000, and, in certain instances, not exceed 16,000. The molecular weight of the copolymer (a) is selected based on the properties that are to be incorporated into the reaction product. The molecular weight of the copolymer may vary in any range of values inclusive of those stated above.

The polydispersity index (PDI) of the copolymer (a) is not always critical. The polydispersity index of the copolymer is usually less than 4, in many cases less than 3.5, typically less than 3.0, and, in some cases, less than 2.5. As used herein, and in the claims, "polydispersity index" is determined from the following equation: (weight average molecular weight (Mw) / number average molecular weight (Mn)). A monodisperse polymer has a PDl of 1.0. Further, as used herein, Mn and Mw are determined from gel permeation chromatography using polystyrene standards.

In an embodiment of the present invention, in the copolymer (a), the alternating sequences of donor monomer - acceptor monomer pairs are residues having the alternating structure IV: 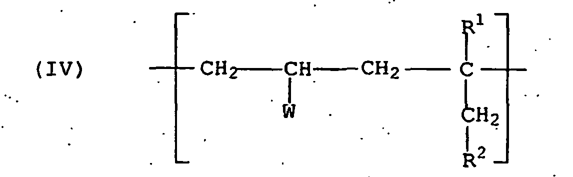 where R¹, R², and W are defined as above. A particularly preferred embodiment is one wherein the monomer residues containing the group W are derived from one or more-acrylic monomers, and the monomer residues containing the groups R¹ and R² are derived from one or a combination of diisobutylene, isobutylene, dipentene, and isoprenol. The copolymer compositions of the present invention may also include other polymerizable, ethylenically unsaturated monomers.

The copolymer (a) may have all of the incorporated monomer residues in an alternating architecture. A non-limiting example of a copolymer segment having 100% alternating architecture of diisobutylene (DIIB) and an acrylic monomer (Ac) is shown by structure V:

(V) -Ac-DIIB-Ac-DIIB-Ac-DIIB-Ac-DIIB-Ac-DIIB-Ac-DIIB-Ac-

However, in most instances, the copolymer will contain alternating segments and random segments as shown by structure VI, a copolymer of DIIB, Ac and other monomers, M:

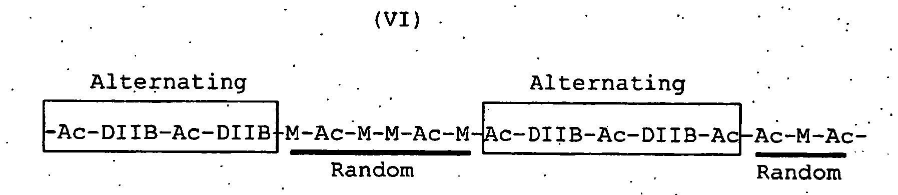

Structure VI shows an embodiment of the present invention where the copolymer may include alternating segments as shown in the boxes and random segments as shown by the underlined segments.

The random segments of the copolymer may contain donor or acceptor monomer residues that have not been incorporated into the copolymer composition by way of an alternating architecture. The random segments of the copolymer composition may further include residues from other ethylenically unsaturated monomers. As recited herein, all references to polymer segments derived from alternating sequences of donor monomer - acceptor monomer pairs are meant to include segments of monomer residues such as those shown by the boxes in structure VI.

The other ethylenically unsaturated monomers include any suitable monomer not traditionally categorized as being an acceptor monomer or a donor monomer.

The other ethylenically unsaturated monomer, residue M of structure VI, is derived from at least one ethylenically unsaturated, radically polymerizable monomer. As used herein and in the claims, "ethylenically unsaturated, radically polymerizable monomer", and like terms, are meant to include vinyl monomers, (meth)acrylic monomers, allylic monomers, olefins, and other ethylenically unsaturated monomers that are radically polymerizable and not classified as donor monomers or acceptor monomers.

Classes of vinyl monomers from which M may be derived include, but are not limited to monomer residues derived from monomers of the general formula VII: 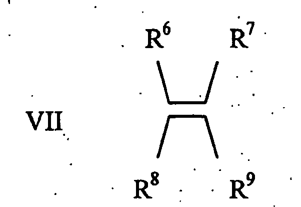 where R⁶, R⁷, and R⁹ are independently selected from the group consisting of H, CF₃, straight or branched alkyl of 1 to 20 carbon atoms, aryl, unsaturated straight or branched alkenyl or alkynyl of 2 to 10 carbon atoms, unsaturated straight or branched alkenyl of 2 to 6 carbon atoms substituted with a halogen, C₃-C₈ cycloalkyl, heterocyclyl and phenyl ; R⁸ is selected from the group consisting of H, C₁-C₆ alkyl, and COOR¹⁰, wherein R¹⁰ is selected from the group consisting of H, an alkali metal, a C₁ to C₆ alkyl group, glycidyl, and aryl.

Specific examples of other monomers, M, that may be used in the present invention include methacrylic monomers and allylic monomers. Residue M may be derived from at least one of alkyl methacrylate having from 1 to 20 carbon atoms in the alkyl group. Specific examples of alkyl methacrylates having from 1 to 20 carbon atoms in the alkyl group from which residue M may be derived include, but are not limited to, methyl methacrylate, ethyl methacrylate, propyl methacrylate, isopropyl methacrylate, butyl methacrylate, isobutyl methacrylate, tert-butyl methacrylate, 2-ethylhexyl methacrylate, lauryl methacrylate, isobornyl methacrylate, cyclohexyl methacrylate, 3,3,5-trimethylcyclohexyl methacrylate, as well as functional methacrylates, such as hydroxyalkyl methacrylates, oxirane functional methacrylates, and carboxylic acid functional methacrylates. Carbamate functional methacrylate monomers are also suitable, such as 2-carbamoyloxyethyl methacrylate, 2-carbamoyloxypropyl methacrylate, and the reaction product of hydroxyethyl methacrylate, isophorone diisocyanate and hydroxypropyl carbamate. Still other carbamate functional ethylenically unsaturated monomers may be used, such as the reaction product of m-TMI and a hydroxyalkyl carbamate.

Residue M may also be selected from monomers having more than one methacrylate group, for example, methacrylic anhydride and diethyleneglycol bis(methacrylate).

As used herein and in the claims, by "allylic monomer(s)" is meant monomers containing substituted and/or unsubstituted allylic functionality, i.e., one or more radicals represented by the following general formula VIII,

(VIII) H₂C=C(R¹¹)-CH₂-

where R¹¹ is hydrogen, halogen, or a C₁ to C₄ alkyl group. Most commonly, R¹¹ is hydrogen or methyl and, consequently, general formula VIII represents the unsubstituted (meth)allyl radical, which encompasses both allyl and methallyl radicals. Examples of allylic monomers include, but are not limited to, (meth)allyl alcohol; (meth)allyl ethers, such as methyl (meth)allyl ether; allyl esters of carboxylic acids, such as (meth)allyl acetate, (meth)allyl butyrate, (meth)allyl 3,4-dimethoxybenzoate, and (meth)allyl benzoate.

The copolymer may be prepared by a method including the steps of (a) providing a donor monomer composition comprising one or more donor monomers of structure I; (b) mixing an ethylenically unsaturated monomer composition comprising one or more acceptor monomers with (a) to form a total monomer composition substantially free of maleate- and fumarate-type monomers; and (c) polymerizing the total monomer composition in the presence of a free radical initiator in the substantial absence of transition metals and Lewis acids. In an embodiment of the present invention, the ethylenically unsaturated monomer composition includes monomers of structure III.

In an embodiment of the present invention, the monomer of structure I is present at a molar excess based on the amount of acrylic acceptor monomer. Any amount of excess monomer of structure I may be used in the making of the copolymer in order to encourage the formation of the desired alternating architecture. The excess amount of monomer of structure I may be at least 10 mol %, in some cases up to 25 mol %, typically up to 50 mol %, and, in some cases, up to 100 mol % based on the amount of acrylic acceptor monomer. When the molar excess of monomer of structure I is too high, the process may not be economical on a commercial scale.

In a further embodiment of the present invention, the acrylic acceptor monomer is present in the copolymer (a) in an amount of at least 15 mol %, in some cases 17.5 mol %, typically at least 20 mol %, and, in some cases, 25 mol % of the total monomer composition. The acrylic acceptor monomer may further be present in an amount up to 50 mol %, in some cases up to 47.5 mol %, typically up to 45 mol %, and, in some cases, up to 40 mol % of the total monomer composition. The level of the acrylic acceptor monomers used is determined by the properties that are to be incorporated into the final reaction product. The acrylic acceptor monomers may be present in the monomer composition in any range of values inclusive of those stated above.

The ethylenically unsaturated monomer composition may include other donor monomers as described above, as well as other monomers designated by M and described above. The use of other mild acceptor monomers is optional. When other mild acceptor monomers are present in the copolymer (a), they are present at a level of at least 0.01 mol % of the copolymer composition, often at least 0.1 mol %, typically at least 1 mol %, and, in some cases, at least 2 mol % of the total monomer composition. The other acceptor monomers may be present at up to 35 mol %, in some cases up to 25 mol %, typically up to 15 mol %, and, in some cases, up to 10 mol % of the total monomer composition. The level of other acceptor monomers used herein is determined by the properties that are to be incorporated into the final reaction product. Residues from the other acceptor monomers may be present in the copolymer (a) in any range of values inclusive of those stated above.

In an embodiment of the present invention, an excess of monomer of structure I is used in the preparation of the copolymer (a) and the unreacted monomer of structure I is removed from the resulting copolymer composition by evaporation. The removal of.unreacted monomer is typically facilitated by the application of a vacuum to the reaction vessel.

Any suitable free radical initiator may be used in the making of the copolymer (a). Examples of suitable free radical initiators include, but are not limited to, thermal free radical initiators, photo-initiators, and redox initiators. Examples of suitable thermal free radical initiators include, but are not limited to, peroxide compounds, azo compounds, and persulfate compounds.

Examples of suitable peroxide compound initiators include, but are not limited to, hydrogen peroxide, methyl ethyl ketone peroxides, benzoyl peroxides, di-t-butyl peroxide, di-t-amyl peroxide, dicumyl peroxide, diacyl peroxides, decanoyl peroxides, lauroyl peroxides, peroxydicarbonates, peroxyesters, dialkyl peroxides, hydroperoxides, peroxyketals, and mixtures thereof.

Examples of suitable azo compounds include, but are not limited to, 4-4'-azobis(4-cyanovaleric acid), 1-1'-azobiscyclohexanecarbonitrile), 2-2'-azobisisobutyronitrile, 2-2'-azobis(2-methylpropionamidine) dihydrochloride, 2-2'-azobis(2-methylbutyronitrile), 2-2'-azobis(propionitrile), 2-2'-azobis(2,4-dimethylvaleronitrile), 2-2'-azobis(valeronitrile), 2,2'-azobis[2-methyl-N-(2-hydroxyethyl)propionamide], 4,4'-azobis(4-cyanopentanoic acid), 2,2'-azobis(N,N'-dimethyleneisobutyramidine), 2,2'-azobis(2-amidinopropane) dihydrochloride, 2,2'-azobis(N,N'-dimethyleneisobutyramidine) dihydrochloride, and 2-(carbamoylazo)-isobutyronitrile.

To prepare the copolymer (a), the ethylenically unsaturated monomer composition and the free radical polymerization initiator may be separately and simultaneously added to and mixed with the donor monomer composition. The ethylenically unsaturated monomer composition and the free radical polymerization initiator may be added to the donor monomer composition over a period of at least 15 minutes, in some cases at least 20 minutes, typically at least 30 minutes, and, in some cases, at least 1 hour. The ethylenically unsaturated monomer composition and the free radical polymerization initiator may further be added to the donor monomer composition over a period of up to 24 hours, in some case up to 18 hours, typically up to 12 hours, and, in some cases, up to 8 hours. The time for adding the ethylenically unsaturated monomer must be sufficient to maintain a suitable excess of donor monomer of structure I over unreacted acrylic acceptor monomer to encourage the formation of donor monomer - acceptor monomer alternating segments. The addition time is not so long as to render the process economically unfeasible on a commercial scale. The addition time may vary in any range of values inclusive of those stated above.

After mixing or during addition and mixing, polymerization of the monomers takes place. The polymerization can be run at any suitable temperature. Suitable temperature for the present method may be ambient, at least 50°C, in many cases at least 60°C, typically at least 75°C, and, in some cases, at least 100°C. Suitable temperature for the present method may further be described as being up to 300°C, in many cases up to 275°C, typically up to 250°C, and, in some cases, up to 225°C. The temperature is typically high enough to encourage good reactivity from the monomers and initiators employed. However, the volatility of the monomers and corresponding partial pressures create a practical upper limit on temperature determined by the pressure rating of the reaction vessel. The polymerization temperature may vary in any range of values inclusive of those stated above.

The polymerization can be run at any suitable pressure. A suitable pressure for the present method may be ambient, at least 1 psi, in many cases at least 5 psi, typically at least 15 psi, and, in some cases, at least 20 psi. Suitable pressures for the present method may further be described as being up to 1000 psi, in many cases up to 600 psi, typically up to 200 psi, and, in some cases, up to 175 psi. The pressure is typically high enough to maintain the monomers and initiators in a liquid phase. The pressures employed have a practical upper limit based on the pressure rating of the reaction vessel employed. The pressure during polymerization temperature may vary in any range of values inclusive of those stated above.

Hydroxyl groups can be introduced into the copolymer (a) directly using a hydroxyl-functional monomer such as hydroxyethyl acrylate in the copolymer, or they can be introduced by functional group transformation. By treating a carboxyl-functional copolymer with an epoxy one can produce a hydroxyl functional polymer. Suitable epoxies include, but are not limited to, ethylene oxide, propylene oxide, butylene oxide and glycidyl neodecanoate.

When the copolymer contains pendent carbamate groups, for example, when using an accepter monomer such as 2-carbamoyloxyethyl(meth)acrylate or 2-carbamoyloxypropyl(meth)acrylate, the carbamate group-containing copolymer can be reacted directly with the aldehyde and monohydric alcohol.

The aldehyde b) most often used in the preparation of the reaction product of the present invention is formaldehyde. Other aldehydes, such as acetaldehyde, propanaldehyde, butyraldehyde, furfural, benzaldehyde, acrolein, methacrolein, and glyoxal are also suitable. The aldehyde b) is used in an amount of 1 to 60 percent by weight, based on the total weight of reactants used to prepare the reaction product. Alkylolation may be performed in an aqueous or alcoholic medium, using techniques known to those skilled in the art; for example, at temperatures of about 10 to about 100°C. in aqueous medium, and about 10 to about 170°C. in organic medium.

Alkylol groups formed during the reaction of a) and b) are at least partially etherified by reaction with at least one monohydric alcohol c). Any monohydric alcohol can be employed for this purpose. Particularly suitable alcohols may have up to 12 carbon atoms, most typically have from 1 to 6 carbon atoms, and include methanol, ethanol, n-propanol, isopropanol, n-butanol, isobutanol, pentanol, hexanol, cyclohexanol, and others, as well as benzyl alcohol and other aromatic alcohols, cyclic alcohols such as cyclohexanol, monoethers of glycols, and halogen-substituted or other substituted alcohols, such as 3-chloropropanol and butoxyethanol. Most commonly, methanol, isobutanol, and/or n-butanol are used.

In the preparation of the reaction product of the present invention, the monohydric alcohol c) is used in an amount of 1 to 70 percent by weight, based on the total weight of reactants used to prepare the reaction product.

When the copolymer a) contains groups that can be converted to carbamate groups, carbamate functional groups may be incorporated into the copolymer a) using any known methods of incorporating carbamate functionality into a polymer. For example, carbamate functional groups may be incorporated into the copolymer a) by reacting terminal functional groups on the copolymer with a carbamate functional material d) via a transcarbamoylation reaction. In this reaction, a low molecular weight carbamate functional material derived from an alcohol or glycol ether is reacted with the functional groups of the copolymer (a), yielding a carbamate functional copolymer and the original alcohol or glycol ether. The low molecular weight carbamate functional material derived from an alcohol or glycol ether can be first prepared by reacting the alcohol or glycol ether with urea in the presence of a catalyst. Suitable alcohols include lower molecular weight aliphatic, cycloaliphatic, and aromatic alcohols such as methanol, ethanol, propanol, butanol, cyclohexanol, 2-ethylhexanol, and 3-methylbutanol. Suitable glycol ethers include ethylene glycol methyl ether and propylene glycol methyl ether. Reactant d) is used in an amount of 1 to 60 percent by weight, based on the total weight of reactants used to prepare the reaction product.

Other carbamate functional materials may be used to convert functional groups on the copolymer to carbamate groups. For example, hydroxyalkyl carbamates including hydroxyethyl carbamate, hydroxypropyl carbamate, hydroxybutyl carbamate, and the like may also be used to transesterify ester groups along the copolymer. Reaction products of ammonia and hydroxyl functional carbonates such as glycerin carbonate or other cyclic carbonates are also suitable.

Alkylolation and etherification of the carbamate functional copolymer may be done in one step by reacting the carbamate functional polymer or oligomer with an aldehyde in an acidic, alcoholic medium such that the alcohol solvent which is in stoichiometric excess participates in the reaction. Alternatively, the alkylolation may first be carried out in a basic aqueous or alcoholic medium. After the alkylolation reaction is complete, the reaction mixture is treated with acid in order to neutralize the base and establish an acidic pH. If the reaction was run under aqueous conditions, the etherifying alcohol can be added to the reaction mixture prior to acidification. The reaction mixture is then typically heated to accomplish the etherification reaction.

In either of the scenarios outlined above, if the etherifying alcohol is immiscible with water the reaction can be heated to reflux and water can be removed via azeotropic distillation in order to drive the equilibrium in favor of the etherification reaction. Partial etherification is possible by stopping the reaction once an amount of water is removed corresponding to the desired degree of etherification. If the alcohol is miscible with water (e.g. methanol) the reaction mixture is simply heated and held until the desired degree of etherification or system equilibrium is reached. If desired, water can be stripped from the reaction mixture with the alcohol once the reaction is complete at a neutral or slightly basic pH to prevent gelling the reaction product.

In a particular embodiment of the invention, the reaction product described above is a copolymer comprising at least 30 mol % of residues having the following alternating structural units: -[DM-AM]-
wherein DM represents a residue from a donor monomer, AM represents a residue from an acceptor monomer, at least 15 mol % of the copolymer comprising a donor monomer having the following structure (I): 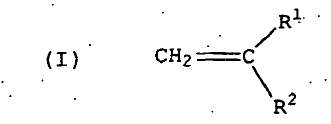 wherein R¹ and R² are as defined above, and the copolymer contains pendant groups of the structure:

-OC(O)N(R")CH₂OR'

where R' is alkyl containing one to eight carbon atoms and R" is selected from H, CH₂OR', linear, cyclic or branched C₁ to C₂₀ alkyl, alkenyl, C₆ to C₂₀ aryl, alkaryl and aralkyl.

The etherified carbamate group-containing copolymer typically will have an equivalent weight of from 125 to 3000, usually from 150 to 600 and often within the range of 200 to 400, based on etherified carbamate groups.

In a separate embodiment of the present invention, a curable composition is provided comprising the reaction product or copolymer described above. The reaction product is capable of self-crosslinking to form a cured product such as a coating. Alternatively, the reaction product or copolymer described above is present as a component in a curable composition comprising:
a) the reaction product described above, typically present in an amount of 1 to 99, often 1 to 50 percent by weight based on the total weight of resin solids in the curable composition; and
b) at least one material having functional groups that are reactive with the reaction product of a), typically present in a total amount of 1 to 99, often 20 to 85 percent by weight based on the total weight of resin solids in the curable composition. The composition of this embodiment is suitable for use as a curable film-forming composition.

The material (b) may comprise reactive functional groups selected from hydroxyl, methylol, methylol ether, carboxylic acid, amide, thiol, urea, carbamate, thiocarbamate, and mixtures thereof. In one embodiment of the present invention, the material (b) comprises carbamate functional groups of the structure: 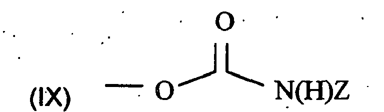 wherein Z is H, or an alkyl or aryl group containing 1 to 12 carbon atoms and may be linear or branched, cyclic, alkaryl or aralkyl and may contain heteroatom substituents.

Functional materials suitable for use as component b) in the curable composition of the present invention may include vinyl polymers, acrylic polymers, polyesters, including alkyds, polyurethanes, polyethers and copolymers and mixtures thereof. As used herein, the term "polymer" is meant to refer to oligomers and both homopolymers and copolymers. Unless stated otherwise, as .used in the specification and the claims, molecular weights are number average molecular weights for polymeric materials indicated as "Mₙ" and obtained by gel permeation chromatography using a polystyrene standard in an art-recognized manner.

Suitable functional polymers for use as component (b) in the curable composition of the present invention include acrylic polymers such as copolymers of one or more alkyl esters of acrylic acid or methacrylic acid, optionally together with one or more other polymerizable ethylenically unsaturated monomers. Useful alkyl esters of acrylic acid or methacrylic acid include aliphatic alkyl esters containing from 1 to 30, and often 4 to 18 carbon atoms in the alkyl group. Non-limiting examples include methyl methacrylate, ethyl methacrylate, butyl methacrylate, ethyl acrylate, butyl acrylate, and 2-ethylhexyl acrylate. Suitable other copolymerizable ethylenically unsaturated monomers include vinyl aromatic compounds such as styrene and vinyl toluene; nitriles such as acrylonitrile and methacrylonitrile; vinyl and vinylidene halides such as vinyl chloride and vinylidene fluoride and vinyl esters such as vinyl acetate.

The acrylic polymer can include hydroxyl functional groups, which are often incorporated into the polymer by including one or more hydroxyl functional monomers in the reactants used to produce the copolymer. Useful hydroxyl functional monomers include hydroxyalkyl acrylates and methacrylates, typically having 2 to 4 carbon atoms in the hydroxyalkyl group, such as hydroxyethyl acrylate, hydroxypropyl acrylate, 4-hydroxybutyl acrylate, hydroxy functional adducts of caprolactone and hydroxyalkyl acrylates, and corresponding methacrylates. The acrylic polymer can be prepared with N-(alkoxymethyl)acrylamides and N-(alkoxymethyl) methacrylamides which result in self-crosslinking acrylic polymers.

Hydroxyl functional groups may be incorporated into the acrylic polymer by using one or more ethylenically unsaturated beta-hydroxy ester functional monomers. Such monomers can be prepared from ethylenically unsaturated, epoxy functional monomers reacted with carboxylic acids having from about 1 to about 20 carbon atoms, often from about 13 to about 20 carbon atoms, or from ethylenically unsaturated acid functional monomers reacted with epoxy compounds containing at least 4 carbon atoms that are not polymerizable with the ethylenically unsaturated acid functional monomer.

Useful ethylenically unsaturated, epoxy functional monomers include glycidyl acrylate, glycidyl methacrylate, allyl glycidyl ether, methallyl glycidyl ether, 1:1 (molar) adducts of ethylenically unsaturated monoisocyanates such as meta-isopropenyl-alpha,alpha-dimethylbenzyl isocyanate with hydroxy functional monoepoxides such as glycidol, and glycidyl esters of polymerizable polycarboxylic acids such as maleic acid, fumaric acid, and crotonic acid. Most often used are the epoxy-functional' acrylates such as glycidyl acrylate, epoxy functional methacrylates such as glycidyl methacrylate, or mixtures thereof. Glycidyl acrylate and glycidyl methacrylate are most often used.

Examples of saturated carboxylic acids include saturated monocarboxylic acids such as those which are noncrystalline at room temperature, particularly those having branched structures. Isostearic acid is most often used. As used herein the term "saturated" as in the phrase "saturated monocarboxylic acid" is intended to denote the absence of ethylenic unsaturation but is not intended to exclude aromatic unsaturation as found, for example, in a benzene ring.

Useful ethylenically unsaturated acid functional monomers include monocarboxylic acids such as acrylic acid, methacrylic acid, crotonic acid; dicarboxylic acids such as itaconic acid, maleic acid and fumaric acid; and monoesters of dicarboxylic acids such as monobutyl maleate and monobutyl itaconate: The ethylenically unsaturated acid functional monomer and epoxy compound are typically reacted in a 1:1 equivalent ratio. The epoxy compound does not contain ethylenic unsaturation that would participate in free radical-initiated polymerization with the unsaturated acid functional monomer. Useful epoxy compounds include 1,2-pentene oxide, styrene oxide and glycidyl esters or ethers, typically containing from 8 to 30 carbon atoms, such as butyl glycidyl ether, octyl glycidyl ether, phenyl glycidyl ether and para-(tertiary butyl) phenyl glycidyl ether. Most often-used glycidyl esters include those of the structure: 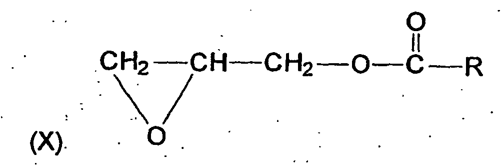 where R is a hydrocarbon radical containing from about 4 to about 26 carbon atoms. Typically, R is a branched hydrocarbon group having from about 8 to about 10 carbon atoms, such as neopentanoate, neoheptanoate or neodecanoate. Suitable glycidyl esters of carboxylic acids include those commercially available from Shell Chemical Company under the trademark CARDURA^{®} E; and from Exxon Chemical Company under the trademark GLYDEXX^{®}-10.

Carbamate functional groups can be included in the acrylic polymer by copolymerizing the acrylic monomers with a carbamate functional vinyl monomer, such as a carbamate functional alkyl ester of methacrylic acid, or by reacting a hydroxyl functional acrylic polymer with a low molecular weight carbamate functional material, such as can be derived from an alcohol or glycol ether, via a transcarbamoylation reaction. Other useful carbamate functional monomers are disclosed in U.S. Patent No. 5,098,947, which is incorporated herein by reference.

Amide functionality may be introduced to the acrylic polymer by using suitably functional monomers in the preparation of the polymer, or by converting other functional groups to amido- groups using techniques known to those skilled in the art. Likewise, other functional groups may be incorporated as desired using suitably functional monomers if available or conversion reactions as necessary.

The acrylic polymer can be prepared by solution polymerization techniques. In conducting the reaction, the monomers are heated, typically in the presence of a free radical initiator such as organic peroxides or azo compounds, for example, benzoyl peroxide or N,N-azobis(isobutyronitrile) and optionally a chain transfer agent, in an organic solvent in which the ingredients as well as the resultant polymer product are compatible. Typically, the organic solvent is charged to a reaction vessel and heated to reflux, optionally under an inert atmosphere. The monomers and other free radical initiator are added slowly to the refluxing reaction mixture. After the addition is complete, some additional initiator may be added and the reaction mixture held at an elevated temperature to complete the reaction.

The acrylic polymer typically has a number average molecular weight of from about 900 to 13,000, often from about 1000 to 5000 as determined by gel permeation chromatography using a polystyrene standard. The acrylic polymers have functional group equivalent weights less than about 5000, often within the range of about 140 to 2500, based on equivalents of reactive functional groups. The term "equivalent weight" is a calculated value based on the relative amounts of the various ingredients used in making the specified material and is based on the solids of the specified material. The rotative amounts are those that result in the theoretical weight in grams of the material, such as a polymer produced from the ingredients, and yield a theoretical number of the particular functional group that is present in the resulting polymer. The theoretical polymer weight is divided by the theoretical number to give the equivalent weight. For example, hydroxyl equivalent weight is based on the equivalents of reactive pendant and/or terminal hydroxyl groups in a hydroxyl-containing polymer.

As discussed above, the functional polymer used in the curable composition of the present invention may alternatively be an alkyd resin or a polyester. Such polymers can be prepared in a known manner by condensation of polyhydric alcohols and polycarboxylic acids. Suitable polyhydric alcohols include but are not limited to ethylene glycol, propylene glycol, butylene glycol, 1,6-hexylene glycol, neopentyl glycol, diethylene glycol, glycerol, trimethylol propane, 2,2,4-trimethyl-1,3-pentanediol, 2,2-dimethyl-3-hydroxypropyl-2,2-dimethyl-3-hydroxypropionate, pentaerythritol, and the like. Suitable polycarboxylic acids include but are not limited to succinic acid, adipic acid, azelaic acid, sebacic acid, maleic acid, fumaric acid, phthalic acid, tetrahydrophthalic acid, hexahydrophthalic acid, methylhexahydrophthalic acid, and trimellitic acid. Besides the polycarboxylic acids mentioned above, functional equivalents of the acids such as anhydrides where they exist or lower alkyl esters of the acids such as methyl esters can be used. Polyesters prepared from polycarboxylic acids and epoxides or polyepoxides as known to those skilled in the art may also be used. Where it is desired to produce air-drying alkyd resins, suitable drying oil fatty acids can be used and include those derived from linseed oil, soya bean oil, tall oil, dehydrated castor oil, or tung oil. The polyesters and alkyd resins can contain a portion of free hydroxyl and/or carboxyl groups that are available for further crosslinking reactions by adjusting the stoichiometry of the reactants used to prepare the polyester or alkyd.

Carbamate functional groups may be incorporated into the polyester by first forming a hydroxyalkyl carbamate which can be reacted with the polyacids and polyols used in forming the polyester. The hydroxyalkyl carbamate is condensed with acid functionality on the polyester, yielding terminal carbamate functionality. Carbamate functional groups may also be incorporated into the polyester by reacting terminal hydroxyl groups on the polyester with a low molecular weight carbamate functional material via a transcarbamoylation process similar to the one described above in connection with the incorporation of carbamate groups into the acrylic polymers, or by reacting isocyanic acid with a hydroxyl functional polyester.

Other functional groups such as amide, thiol, urea, and thiocarbamate may be incorporated into the polyester or alkyd resin as desired using suitably functional reactants if available, or conversion reactions as necessary to yield the desired functional groups. Such techniques are known to those skilled in the art.

The polyester polymer typically has a number average molecular weight of from about 600 to 3000, often from about 800 to 1500 as determined by gel permeation chromatography using a polystyrene standard, and a functional group equivalent weight within the range of about 200 to 1500, often about 300 to 400, based on equivalents of reactive pendant or terminal functional groups.

Polyurethanes can also be used as the functional compound in the curable composition. Useful polyurethanes include polymeric polyols which are prepared by reacting polyhydric alcohols, polyester polyols or acrylic polyols, such as those mentioned above or polyether polyols, such as those mentioned below with a polyisocyanate such that the OH/NCO equivalent ratio is greater than 1:1 so that free hydroxyl groups are present in the product. Alternatively, isocyanate functional polyurethanes may be prepared using similar reactants in relative amounts such that the OH/NCO equivalent ratio is less than 1:1, and the isocyanate functional polyurethanes may be modified to contain functional groups that are reactive with the reaction product or composition of matter of a).

The organic polyisocyanate that is used to prepare the polyurethane polymer can be an aliphatic or aromatic polyisocyanate or mixtures thereof. Diisocyanates are most often used, although higher polyisocyanates can be used in place of or in combination with diisocyanates. Examples of suitable aromatic diisocyanates include 4,4'-diphenylmethane diisocyanate and toluene diisocyanate. Examples of suitable aliphatic diisocyanates include straight chain aliphatic diisocyanates such as 1,6-hexamethylene diisocyanate. Also, cycloaliphatic diisocyanates such as isophorone diisocyanate and 4,4',-methylene-bis-(cyclohexyl isocyanate) can be used. Examples of suitable higher polyisocyanates include 1,2,4-benzene triisocyanate and polymethylene polyphenyl isocyanate. Additional polyisocyanates such as those disclosed above in the preparation of the reaction product of the present invention may also be used.

Terminal and/or pendent carbamate functional groups can be incorporated into the polyurethane by reacting a polyisocyanate with a polymeric polyol containing the terminal/pendent carbamate groups. Alternatively, carbamate functional groups can be incorporated into the polyurethane by reacting a polyisocyanate with a polyol and a hydroxyalkyl carbamate or isocyanic acid as separate reactants. Carbamate functional groups can also be incorporated into the polyurethane by reacting a hydroxyl functional polyurethane with a low molecular weight carbamate functional material via a transcarbamoylation process similar to the one described above in connection with the incorporation of carbamate groups into the acrylic polymer. Additionally, an isocyanate functional polyurethane can be reacted with a hydroxyalkyl carbamate to yield a carbamate functional polyurethane.

Other functional groups such as amide, thiol, urea, and thiocarbamate may be incorporated into the polyurethane as desired using suitably functional reactants if available, or conversion reactions as necessary to yield the desired functional groups. Such techniques are known to those skilled in the art.

The polyurethane typically has a number average molecular weight of from about 600 to 3000, often from about 800 to 1500 as determined by gel permeation chromatography using a polystyrene standard. The polyurethanes typically have functional group equivalent weights within the range of about 200 to 1500, based on equivalents of reactive functional groups.

Examples of polyether polymers useful in the curable composition of the present invention are polyalkylene ether polyols including those having the following structural formula: 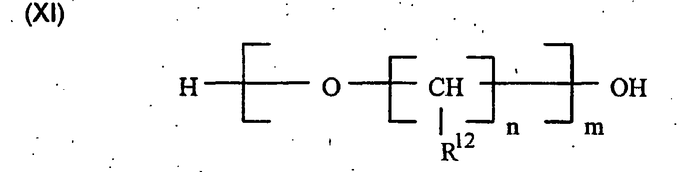 or 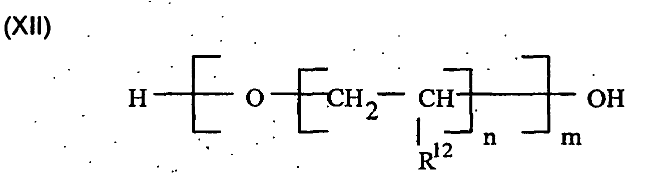 where the substituent R¹² is hydrogen or lower alkyl containing from 1 to 5 carbon atoms including mixed substituents, and n is typically from 2 to 6 and m is from 8 to 100 or higher. Included are poly(oxytetramethylene) glycols, poly(oxytetraethylene) glycols, poly(oxy-1,2-propylene) glycols, and poly(oxy-1,2-butylene) glycols.

Also useful are polyether polyols formed from oxyalkylation of various polyols, for example, diols such as ethylene glycol, 1,6-hexanediol, Bisphenol A and the like, or other higher polyols such as trimethylolpropane, pentaerythritol, and the like. Polyols of higher functionality that can be utilized as indicated can be made, for instance, by oxyalkylation of compounds such as sucrose or sorbitol. One commonly utilized oxyalkylation method is reaction of a polyol with an alkylene oxide, for example, propylene or ethylene oxide, or other cyclic ether such as tetrahydrofuran, in the presence of an acidic or basic catalyst. Polyethers used most often include those sold under the names PLURACOL, TERATHANE and TERACOL, available from BASF, and E. I. Du Pont de Nemours and Company, Inc., respectively, and POLYMEG, available from Q O Chemicals, Inc., a subsidiary of Great Lakes Chemical Corp.

Most often, pendant or terminal carbamate functional groups may be incorporated into the polyethers by a transcarbamoylation reaction as described above.

Other functional groups such as amide, thiol, urea, and thiocarbamate may be incorporated into the polyether as desired using suitably functional reactants if available, or conversion reactions as necessary to yield the desired functional groups. The polyether polymer typically has a number average molecular weight of from about 500 to 5000, more typically from about 900 to 3200 as determined by gel permeation chromatography using a polystyrene standard, and an equivalent weight of within the range of 140 to 2500, often about 500, based on equivalents of reactive pendant or terminal functional groups.

The curable composition may further include one or more auxiliary crosslinking agents such as free and/or capped polyisocyanates; triazine compounds of the formula: C₃N₃(NHCOXR¹³)₃, wherein X is nitrogen, oxygen, sulfur, phosphorus, or carbon, and R¹³ is a lower alkyl group having one to twelve carbon atoms, or mixtures of lower alkyl groups; and conventional aminoplast crosslinking agents.

Suitable polyisocyanates include any of those disclosed above. Any suitable aliphatic, cycloaliphatic, or aromatic alkyl monoalcohol or phenolic compound may be used as a capping agent for the polyisocyanate. Examples include lower aliphatic alcohols such as methanol, ethanol, and n-butanol; cycloaliphatic alcohols such as cyclohexanol; aromatic-alkyl alcohols such as phenyl carbinol and methylphenyl carbinol; and phenolic compounds such as phenol itself and substituted phenols wherein the substituents do not affect coating operations, such as cresol and nitrophenol. Glycol ethers may also be used as capping agents. Suitable glycol ethers include ethylene glycol butyl ether, diethylene glycol butyl ether, ethylene glycol methyl ether and propylene glycol methyl ether.

Other suitable capping agents include pyrazoles such as 3,5-dimethyl pyrazote, oximes such as methyl ethyl ketoxime, acetone oxime and cyclohexanone oxime, lactams such as epsilon-caprolactam, and secondary amines such as dibutyl amine.

Triazine compounds of the type mentioned are described in U.S. Patent No. 4,939,213, incorporated herein by reference.

Conventional aminoplast crosslinking agents are well known in the art and are described in U.S. Pat. No. 5,256,452; Col. 9, Lines 10-28. Useful aminoplast resins are based on the addition products of formaldehyde with an amino- or amido-group carrying substance. Condensation products obtained from the reaction of alcohols and formaldehyde with melamine, urea or benzoguanamine are most common and most often used herein. While the aldehyde employed is most often formaldehyde, other similar condensation products can be made from other aldehydes, such as acetaldehyde, crotonaldehyde, acrolein, benzaldehyde, furfural, glyoxal and the like.

Condensation products of other amines and amides can also be used, for example, aldehyde condensates of triazines, diazines, triazoles, guanadines, guanamines and alkyl- and aryl-substituted derivatives of such compounds, including alkyl- and aryl-substituted ureas and alkyl- and aryl-substituted melamines. Non-limiting examples of such compounds include N,N'-dimethyl urea, benzourea, dicyandiamide, formaguanamine, acetoguanamine, glycoluril, ammeline, 3,5-diaminotriazole, triaminopyrimidine, and 2-mercapto-4,6-diaminopyrimidine. The aminoplast crosslinking agent may be monomeric or polymeric and may be partially or fully alkylated.

Generally, the auxiliary crosslinking agent is present in the curable composition of the present invention in an amount ranging from about 0 to about 50 weight percent on a basis of total resin solids of the curable composition, often about 5 to about 40 weight percent.

In each embodiment of the present invention, the curable compositions may optionally contain at least one other polymer separate from and in addition to the functional group-containing compound(s) that are reactive with the reaction product and any auxiliary crosslinking agent(s). The additional polymer(s) may or may not contain functional groups and may be selected from acrylic polymers, polyester polymers, which are most often used, polyurethane polymers, polyether polymers, polysiloxane -polymers, polyolefins and mixtures thereof. These polymers are often hydroxyl or carbamate functional and may be prepared as discussed above. Other functional groups include epoxide, silane, carboxylic acid, anhydride, and the like.

When the curable compositions of the present invention contain additional functional group-containing polymers, the additional polymers are present in total amounts up to 50, often about 5 to 35, more often 5 to 20 percent by weight based on the total weight of resin solids in the curable composition.

In each embodiment of the present invention, the curable composition may further include one or more auxiliary crosslinking agents as disclosed above, such as free and/or capped polyisocyanates; triazine compounds of the formula: C₃N₃(NHCOXR)₃, wherein X is nitrogen, oxygen, sulfur, phosphorus, or carbon, and R is a lower alkyl group having one to twelve carbon atoms, or mixtures of lower alkyl groups; and conventional aminoplast crosslinking agents.

Other optional ingredients, such as catalysts, plasticizers, antioxidants, thixotropic agents, hindered amine light stabilizers, UV light absorbers and stabilizers may be formulated into the curable compositions of the present invention. These ingredients may be present (on an individual basis) in amounts up to 10 percent, often from about 0.1 to 5 percent by weight based on total weight of resin solids of the curable composition. Suitable catalysts include acid functional catalysts known to those skilled in the art as useful in aminoplast-cured compositions, such as phenyl acid phosphate, para-toluenesulfonic acid, dodecylbenzene sulfonic acid, and the like.

The curable compositions of the present invention may be used as curable film-forming compositions and may contain color pigments conventionally used in surface coatings and may be used as high gloss monocoats; that is, high gloss pigmented coatings. By "high gloss" it is meant that the cured coating has a 20° gloss and/or a DOI ("distinctness of image") measurement of at least about 80 as measured by standard techniques known to those skilled in the art. Such standard techniques include ASTM D523 for gloss measurement and ASTM E430 for DOI measurement.

The curable compositions of the present invention are often used as clear coats in multi-component composite coating compositions such as color-plus-clear composite coating compositions. A color-plus-clear composition typically comprises a base coat deposited from a pigmented or colored film-forming composition, and a transparent topcoat (clear coat) applied over the base coat.

The multi-component composite coating compositions can be applied to various substrates to which they adhere, including wood, metals, glass, cloth, polymeric substrates and the like. They are particularly useful for coating metals and elastomeric substrates that are found on motor vehicles. The compositions can be applied by conventional means including brushing, dipping, flow coating, spraying and the like, but they are most often applied by spraying. The usual spray techniques and equipment for air spraying and electrostatic spraying and either manual or automatic methods can be used.

First, a base coat composition is applied to the surface of the substrate to be coated. The base coat composition can be waterborne, solventborne or powdered, and typically includes a film-forming resin, crosslinking material (such as are discussed above) and pigment. Non-limiting examples of suitable base coat compositions include waterborne base coats for color-plus-clear composites such as are disclosed in U.S. Patent Nos. 4,403,003; 4,147,679; and 5,071,904, each of which is incorporated by reference herein.

After application of the base coat to the substrate, there is typically a drying or flash-off period allowed prior to the application of the clear coat. The purpose of this period is to evaporate at least a portion of the solvent or water from the base coat film. The flash-off conditions may vary by time, temperature, and/or humidity, depending on the particular base coat composition, the desired appearance and properties of the final film. Typical times are from 1 to 15 minutes at a temperature between 70 and 250°F (21.1 and 121.1°C.) More than one base coat layer and multiple topcoat layers may be applied to the substrate to develop optimum appearance. Typically, the base coat thickness ranges from about 0.1 to about 5 mils (about 2.54 to about 127 microns), and often about 0.4 to about 1.5 mils (about 10.1.6 to about 38.1 microns) in thickness.

After application of the base coat, the topcoat described in detail above is applied. The topcoat coating composition can be applied to the surface of the base-coat by any of the coating processes discussed above for applying the base coat coating composition to the substrate. The coated substrate is then heated to cure the coating layers. In the curing operation, solvents are driven off and the film-forming materials of the clear coat and the base coat are each crosslinked. The heating or curing operation is usually carried out at a temperature in the range of from 160-350°F (71-177°C) but if needed, lower or higher temperatures may be used as necessary to activate crosslinking mechanisms. The thickness of the clear coat usually ranges from about 0.5 to about 5 mils (about 12.7 to about 127 microns), often about 1.0 to about 3 mils (about 25.4 to about 76.2 microns).

As used herein, the term "cure" as used in connection with a composition, e.g., "a curable composition," shall mean that any crosslinkable components of the composition are at least partially crosslinked. In certain embodiments of the present invention, the crosslink density of the crosslinkable components, i.e., the degree of crosslinking, ranges from 5% to 100% of complete crosslinking. In other embodiments, the crosslink density ranges from 35% to 85% of full crosslinking. In other embodiments, the crosslink density ranges from 50% to 85% of full crosslinking. One skilled in the art will understand that the presence and degree of crosslinking, i.e., the crosslink density, can be determined by a variety of methods, such as dynamic mechanical thermal analysis (DMTA) using a Polymer Laboratories MK III DMTA analyzer conducted under nitrogen. This method determines the glass transition temperature and crosslink density of free films of coatings or polymers. Another common test for determining the presence of crosslinking is solvent double-rubs, such as with methyl ethyl ketone. These physical properties of a cured material are related to the structure of the crosslinked network.

According to the DMTA method, the length, width, and thickness of a sample to be analyzed are first measured, the sample is tightly mounted to the Polymer Laboratories MK III apparatus, and the dimensional measurements are entered into the apparatus. A thermal scan is run at a heating rate of 3°C/min, a frequency of 1 Hz, a strain of 120%, and a static force of 0.01 N, and sample measurements occur every two seconds. The mode of deformation, glass transition temperature, and crosslink density of the sample can be determined according to this method. Higher crosslink density values indicate a higher degree of crosslinking in the coating.

The present invention will further be described by reference to the following examples. The following examples are merely illustrative of the invention and are not intended to be limiting. Unless otherwise indicated, all parts are by weight.

### EXAMPLE A

A diisobutylene /4-hydroxybutyl acrylate /butyl acrylate copolymer was prepared according to the following procedure:

| | Ingredients | Parts by weight (grams) |
|---|---|---|
| Charge 1 | Diisobutylene | 4480.00 |
| | DOWANOL PM¹ | 200.00 |
| Charge 2 | t-Amylperoxy (2-ethyl hexanoate) | 144.00 |
| Charge 3 | 4-Hydroxybutyl acrylate | 432.00 |
| | Butyl acrylate | 384.00 |

| | | |
|---|---|---|
| ¹ 1-methoxy-2-propanol, available from Dow Chemical. | | |

Charge 1 was added a reaction flask equipped with an agitator, a thermocouple, and a N₂ inlet, placed under blanket of N₂, and heated to 103°C. Charge 2 was added to the reactor over 3.5 hours. After 15 minutes Charge 3 was added to the reactor over 3 hours. During the monomer addition the temperature was maintained at 103°C. After Charges 2 and 3 were in the reactor, the reaction mixture was held for 2 hours. The reactor was then cooled to 25°C. GC analysis of the reaction mixture showed that all acrylates were reacted. The reaction flask was then equipped for simple vacuum distillation, and the reaction mixture heated to 80°C to remove the unreacted diisobutylene and the solvent. The solids content of the resulting polymer was determined to be 94.22 % (110°C for 1 hour). The copolymer had a number average molecular weight (Mₙ) of 1710 and polydispersity M_{w}/Mₙ of 1.9 (determined by gel permeation chromatography using polystyrene as a standard). The NMR spectrum is consistent with copolymer composition 42.60% diisobutylene, 28.70% 4-hydroxybutyl acrylate and 28.70% butyl acrylate.

### EXAMPLE B

A diisobutylene /4-hydroxybutyl acrylate / butyl acrylate copolymer was carbamoylated according to the following procedure:

| | Ingredients | Parts by weight (grams) |
|---|---|---|
| Charge 1 | DIB /4-HBA / BA resin of Example A | 532.0 |
| | Methyl carbamate | 75.0 |
| | DOWANOL PM | 135.0 |
| | Butyl stannoic acid | 1.15 |
| | Triphenylphosphite | 1.15 |
| Charge 2 | 2-methyl-1-propanol | 182.0 |

Charge 1 was added to a reaction flask equipped with a thermocouple, an overhead stirrer, a N₂ inlet, a short fractionating column packed with ceramic saddles, a distillation head equipped with a thermocouple, a condenser, and a distillate receiver. The reaction mixture was heated between 143° and 154°C, during which time 69 g of distillate were collected in the receiver. During the distillation, care was taken to keep the distillation head temperature below 70°C. When distillate stopped coming over at 154°C, the reaction mixture was cooled to 140°C and the flask was equipped for vacuum distillation. At this temperature the pressure of the flask was gradually reduced with removal of distillate. When a pressure of 60 mm Hg was attained, the temperature of the reaction mixture was raised to 150°C and held until no more distillate came over. The vacuum was broken, the reaction mixture was sampled, and Charge 2 was added to the reaction flask. Prior to the addition of Charge 2, the reaction product was found to have an OH value of 21.7. The resulting polymer solution had a measured solids of 72.3% (110°C, 1hr), a Gardner-Holt bubble tube viscosity of U, a M_{w} of 3048 and a Mₙ of 1385 as determined by gel permeation chromatography vs. a polystyrene standard.

### EXAMPLE C

An aminoplast based on a carbamoylated DIB / 4-hydroxybutyl acrylate / butyl acrylate copolymer was prepared according to the following procedure:

| | Ingredients | Parts by weight (grams) |
|---|---|---|
| Charge 1 | Carbamoylated DIB / 4-HPA / BA resin of Example B | 327.9 |
| | 2-methyl-1-propanol | 133.2 |
| | 53% n-BuOH / 40% formaldehyde solution | 67.5 |
| | Phosphorous acid | 2.52 |

Charge 1 was added to a reaction flask equipped with a thermocouple, an overhead stirrer, a N₂ inlet, a condenser, and a Dean-Stark trap primed with 2-methyl-1-propanol. The reaction mixture was heated to reflux (102°C), at which time H₂O began to be collected at the bottom of the Dean-Stark trap. When 4 g of H₂O had been removed, an additional 2.52 g of phosphorous acid were added to the reaction mixture. The temperature of the reaction mixture was gradually increased to 113°C, at which time no additional H₂O was being evolved. The total amount of H₂O collected was 14 g. The resulting polymer solution had a measured solids of 53.1% (110°C, 1 hr), a Gardner-Holt bubble tube viscosity of B, an acid value of 12, and an M_{w} of 5272 and an Mₙ of 1712 as determined by gel permeation chromatography vs. a polystyrene standard.

### EXAMPLE D

A diisobutylene / hydroxypropyl acrylate copolymer was prepared according to the following procedure:

| | Ingredients | Parts by weight (grams) |
|---|---|---|
| Charge 1 | Diisobutylene | 1750.0 |
| | Isopropanol | 550.0 |
| | | |
| Charge 2 | Di-t-amyl peroxide | 150.0 |
| | 2-methyl-1-propanol | 50 |
| | | |
| Charge 3 | Hydroxypropyl acrylate | 1250.0 |

Charge 1 was added to a suitable reactor and purged with N₂. A 5 psig pad was left on the reactor. The reaction mixture was heated to 150.9°C. At this point the reactor pressure was 101.4 psig. Charge 2 was begun over 2.5 hours at a rate of 80 g / hour. Fifteen minutes after the beginning of Charge 2, Charge 3 was begun over 2 hours. After the completion of Charge 2 the reaction mixture was held at temperature for two hours, cooled to < 30°C, and filled out to a suitable 5L vessel that was equipped for atmospheric distillation. Isopropanol and excess diisobutylene were then removed from the reaction mixture at a temperature of 125°C. 1684 g of distillate was collected. The vessel was then switched to vacuum distillation at a maximum temperature of 140°C; an additional 210 g distillate were collected. The reaction mixture was held an additional 30 minutes at 140°C prior to cooling and fill-out. The resulting polymer had a measured solids of 92.9% (110°C / 1 hour), a residual isopropanol content of <0.01 % by gas chromatography; an OH value of 307.4, a weight average molecular weight of 1519, and a number average molecular weight of 916 as determined by gel permeation chromatography versus a polystyrene standard.

### EXAMPLE E

A diisobutylene / hydroXypropyl acrylate copolymer was carbamoylated according to the following procedure:

| | Ingredients | Parts by weight (grams) |
|---|---|---|
| Charge 1 | DIB / HPA resin of Example D | 547.5 |
| | Methyl carbamate | 225.0 |
| | DOWANOL PM | 405.0 |
| | Butyl stannoic acid | 3.45 |
| | Triphenylphosphite | 3.45 |

Charge 1 was added to a reaction vessel equipped with a thermocouple, an overhead stirrer, a N₂ inlet, and a reflux condenser. The reaction mixture was heated to reflux (137°C) and held for 1 hour. The reaction mixture was then cooled to slightly below reflux temperature, and the reflux condenser was removed and the vessel equipped for fractional atmospheric distillation (a short fractionating column packed with ceramic saddles, a distillation head equipped with a thermocouple, a condenser, and a distillate receiver). The reaction mixture was reheated to a maximum temperature of 150°C and 391 g of distillate was collected. The reaction vessel was then equipped for vacuum distillation. At a temperature of 140°C, the pressure of the flask was gradually reduced; at the maximum attainable vacuum, the temperature was raised to 159°C to complete the distillation. The resulting polymer solution had a measured solids of 93.0% (110°C, 1 hour), and a OH value of 71.

### EXAMPLE F

A partially carbamoylated diisobutylene / hydroxypropyl acrylate copolymer was further carbamoylated according to the following procedure:

| | Ingredients | Parts by weight (grams) |
|---|---|---|
| Charge 1 | Resin of Example E | 644.3 |
| | Methyl carbamate | 56.3 |
| | DOWANOL PM | 135.0 |
| | Butyl stannoic acid | 1.95 |
| | Triphenylphosphite | 3.45 |
| | | |
| Charge 2 | 2-methyl-1-propanol | 546.0 |

Charge 1 was added to a reaction vessel equipped with a thermocouple, an overhead stirrer, a N₂ inlet, and a reflux condenser. The reaction mixture was heated to reflux (149°C) and held for 1 hour. The reaction mixture was then cooled to slightly below reflux temperature, and the reflux condenser was removed and the vessel equipped for fractional atmospheric distillation (a short fractionating column packed with ceramic saddles, a distillation head equipped with a thermocouple, a condenser, and a distillate receiver). The reaction mixture was reheated to a maximum temperature of 170°C and held until no additional distillate came off. The reaction vessel was then equipped for vacuum distillation. At a temperature of 140°C, the pressure of the flask was gradually reduced; at the maximum attainable vacuum, the temperature was raised to 143°C to complete the distillation. The reaction mixture was then thinned with Charge 2. The polymer prior to the addition of Charge 2 was found to have a OH value of 49.5. After thinning, the polymer solution had a measured solids of 48.7% (110°C / 1 hour), a weight average molecular weight of 2333, and a number average molecular weight of 1022 as determined by gel permeation chromatography versus a polystyrene standard.

### EXAMPLE G

An aminoplast based on a carbamoylated DIB / hydroxypropyl acrylate copolymer was prepared according to the following procedure:

| | Ingredients | Weight in grams |
|---|---|---|
| Charge 1 | Carbamoylated DIB / HPA resin of Example F | 240.6 |
| | 2-methyl-1-propanol | 189.1 |
| | 53% n-BuOH / 40% formaldehyde solution | 75.0 |
| | Phosphorous acid | 5.0 |

Charge 1 was added to a reaction flask equipped with a thermocouple; an overhead stirrer, a N₂ inlet, a condenser, and a Dean-Stark trap primed with 2-methyl-1-propanol. The reaction mixture was heated to reflux (102°C), at which time H₂O began to be collected at the bottom of the Dean-Stark trap. When 14 g of H₂O had been removed, an additional 2.5 g of phosphorous acid were added to the reaction mixture. The temperature of the reaction mixture was gradually increased to 108°C, at which time no additional H₂O was being evolved. The total amount of H₂O collected was 17 g. The resulting polymer solution had a measured solids of 31 % (110°C, 1hr), a M_{w} of 2873 and a Mₙ of 1178 as determined by gel permeation chromatography vs. a polystyrene standard.

### EXAMPLE H

An isobutylene / hydroxyethyl acrylate / methyl acrylate copolymer was prepared according to the following procedure:

| | Ingredients | Wt in grams |
|---|---|---|
| Charge 1 | Isopropanol | 540.0 |
| | | |
| Charge 2 | Di-t-amyl peroxide | 60.0 |
| | Isopropanol | 60.0 |
| | | |
| Charge 3 | Isobutylene | 500.0 |
| | | |
| Charge 4 | Methyl acrylate | 640.0 |
| | Hydroxyethyl acrylate | 860.0 |
| Charge 5 | Solvesso 100 | 450.0 |

Charge 1 was added a reaction flask equipped with an agitator, a thermocouple, and a N₂ inlet, placed under a 5 psig N₂ pad, and heated to 1,57°C with an agitation rate of 500 rpm. Charge 2 was started and added to reactor over 2.5 hours. Fifteen minutes after the beginning of Charge 2, Charge 3 and Charge 4 were begun and added to the reactor over 2 hours; the reactor temperature at the beginning of the addition was 164°C. At the conclusion of the charges, the reactor temperature was 169°C. the reaction mixture was then held for 2 hours. The reaction mixture was then cooled to 40°C, transferred to a flask was than equipped for total distillation, and heated to 155°C. At that temperature, full vacuum was then applied to the system for 30 minutes. The batch was then cooled to 125°C, and Charge 5 was added. The resulting polymer solution had a OH value of 177.1, a measured solids of 78.4% (110°C. 1hr). and an M_{w} of 2558 and an Mₙ of 1170 as determined by gel permeation chromatography vs. a polystyrene standard.

### EXAMPLE I

An isobutylene hydroxyethyl acrylate /methyl acrylate copolymer was carbamoylated according to the following procedure:

| | Ingredients | Parts by weight (grams) |
|---|---|---|
| Charge 1 | IB / HEA / MA resin of Example H | 950.4 |
| | | |
| Charge 2 | DOWANOL PM | 405.0 |
| | | |
| Charge 3 | Methyl carbamate | 225.0 |
| | Butyl stannoic acid | 1.9 |
| | Triphenylphosphite | 1.9 |
| | | |
| Charge 4 | 2-methyt-1-propanol | 666.0 |

Charge 1 was added to a reaction flask equipped with a thermocouple, an overhead stirrer, a N₂ inlet, a distillation head equipped with a thermocouple, a vacuum inlet, a condenser, and a distillate receiver. The reactor contents were heated to 165°C and distillate was removed from the reaction mixture. As the distillation slowed, vacuum was applied to the reactor to complete the distillation. The reactor was then equipped for reflux and Charge 2 was added, followed by Charge 3. The reaction mixture was heated to reflux and held for 90 minutes, then cooled to below reflux temperature. The reactor was then equipped with a short fractionating column, still head, thermocouple, and receiver. The reaction mixture was then heated to a temperature at which distillate began to come over (133°C). During the distillation, care was taken to keep the distillation head temperature below 70°C. When distillate stopped coming over at 155°C the flask was equipped for vacuum distillation. At this temperature the pressure of the flask was gradually reduced with removal of distillate. The vacuum was broken, the reaction mixture was sampled, and Charge 4 was added to the reaction flask. Prior to the addition of Charge 4, the reaction product was found to have a residual OH value of 67.7. The resulting polymer solution had a measured solids of 54.0% (110°C, 1 hr), a Gardner-Holt bubble tube viscosity of U+, and a M_{w} of 10743 and a Mₙ of 2164 as determined by gel permeation chromatography vs. a polystyrene standard.

### EXAMPLE J

An aminoplast based on a carbamoylated IB / hydroxyethyl acrylate / methyl acrylate copolymer was prepared according to the following procedure:

| | Ingredients | Parts by weight (grams) |
|---|---|---|
| Charge 1 | Carbamoylated IB / HEA / MA resin of Example I | 514.6 |
| | 2-methyl-1-propanol | 263.4 |
| | 53% n-BuOH / 40% formaldehyde solution | 150.0 |
| | Phosphorous acid | 10.0 |

Charge 1 was added to a reaction flask equipped with a thermocouple, an overhead stirrer, a N₂ inlet, a condenser, and a Dean-Stark trap primed with 2-methyl-1-propanol. The reaction mixture was heated to reflux (104°C), at which time H₂O began to be collected at the bottom of the Dean-Stark trap. When 1 g of H₂O had been removed, an additional 2 g of phosphorous acid were added to the reaction mixture. When 1 g of H₂O had been removed, another 3 g of phosphorous acid were added to the reaction mixture. The temperature of the reaction mixture was gradually increased to 110°C, at which time no additional H₂O was being evolved. The total amount of H₂O collected was 37 g. The resulting polymer solution had a measured solids of 35.5% (110°C, 1hr), a Gardner-Holt bubble tube viscosity of < A, and an M_{w} of 9447 and an Mₙ of 2007 as determined by gel permeation chromatography vs. a polystyrene standard.

### FORMULATION EXAMPLES 1-5

The following coating compositions were prepared:

| | Weight in grams | | | | |
|---|---|---|---|---|---|
| Ingredient | Ex 1 (comp) | Ex 2 (comp) | Ex 3 | Ex 4 | Ex 5 |
| Carbamoylated DIB / 4-HBA / BA resin of example B | 20.2 | | | | |
| Carbamoylated IB HEA / MA resin of Example H | | 50.0 | | | |
| DIB / 4-HBA / BA carbamate aminoplast resin of Example C | | | 20.2 | | |
| IB / HEA / MA carbamate aminoplast of Example J | | | | 50.0 | |
| DIB / HPA carbamate aminoplast of Example G | | | | | 10.0 |
| Dodecylbenzenesulfonic acid (70% solution in isopropanol) | 0.21 | 0.41 | 0.16 | 0.35 | 0.04 |

The coating compositions were drawn down on steel panels coated with an electrodeposition primer, allowed to flash 10 minutes, and baked in an oven for 30 minutes at 140°C. The cure response was evaluated via methyl ethyl ketone (MEK) double rubs. The results are summarized in the following table:

| Example | MEK resistance (double rubs) | Comments |
|---|---|---|
| 1 | < 5 | Very tacky to touch |
| 2 | 3 | Very tacky to touch |
| 3 | 100 | Not tacky; scrapable with fingernail at site of MEK rubs immediately after test |
| 4 | >100 | Not tacky; scrapable with fingernail at site of MEK rubs immediately after test |
| 5 | >100 | No mar |

From the above data, it is clear that the aminoplast modified carbamate resins exhibit crosslinking compared to the unmodified controls.

### FORMULATION EXAMPLES 6-10

The following coating compositions were prepared:

| | Weight in grams | | | | |
|---|---|---|---|---|---|
| | Ex 6 | Ex 7 | Ex 8 | Ex 9 | Ex 10 |
| Methyl n-amyl ketone | 3.63 | 3.63 | 3.63 | 3.63 | 1.82 |
| Xylene | 1.10 | 1.10 | 1.10 | 1.10 | 0.55 |
| SOLVESSO 100¹ | 3.74 | 3.74 | 3.74 | 3.74 | 1.87 |
| Ethylene glycol monohexyl ether | 0.36 | 0.36 | 0.36 | 0.36 | 0.18 |
| Ethanol | 1.66 | 1.66 | 1.66 | 1.66 | 0.83 |
| Acrylic polyol² | 50.00 | 42.86 | 35.71 | 46.4 | 23.2 |
| DIB / HPA carbamate aminoplast of Example G | 48.08 | 64.10 | 80.13 | 48.08 | 24.04 |
| Cymel 303³ | | | | 5.0 | |
| Cymel 1130⁴ | | | | | 2.5 |
| DDBSA (70% solution in isopropyl alcohol) | 0.71 | 0.71 | 0.71 | 0.71 | 0.35 |

| | | | | | |
|---|---|---|---|---|---|
| ¹ Mixed aromatic solvent available from Exxon Corp. ² Acrylic polyol comprising 39.5% hydroxypropyl acrylate, 19.8% styrene, 18.8% butyl acrylate, 18.3% butyl acrylate, 2.2% acrylic acid, 1.0% t-dodecane thiol, 71.3% solids in 45.8% SOLVESSO 100, 44.2% xylene, 10.0% isobutyl alcohol, available from PPG Industries, Inc. ³ Methylated melamine formaldehyde resin available from CYTEC Industries, Inc. ⁴ Methylated, butylated melamine formaldehyde resin available from CYTEC Industries, Inc. | | | | | |

The coating compositions were drawn down on steel panels coated with an electrodeposition primer, allowed to flash 10 minutes, and baked in an oven for 30 minutes at 140°C. The cure response was evaluated via methyl ethyl ketone (MEK) double rubs. The results are summarized in the following table:

| Example | MEK resistance (double rubs) | Comments |
|---|---|---|
| 6 | > 100 | Mars at 10 double rubs |
| 7 | > 100 | Mars at 10 double rubs |
| 8 | > 100 | Mars at 10 double rubs |
| 9 | > 100 | No mar |
| 10 | > 100 | Very slight mar |

The examples show that all the films are cured. Adding a small amount of melamine to the coating composition improves the cure response.

It will be appreciated by those skilled in the art that changes could be made to the embodiments described above without departing from the broad inventive concept thereof. It is understood, therefore, that this invention is not limited to the particular embodiments disclosed, but it is intended to cover modifications that are within the spirit and scope of the invention, as defined by the appended claims.

## Claims

1. A compound that is the reaction product of reactants, wherein the reactants comprise:
a) at least one copolymer comprising at least 30 mol % of residues having the following alternating structural units:
-[DM-AM]-
wherein DM represents a residue from a donor monomer, AM represents a residue from an acceptor monomer, at least 15 mol % of the copolymer comprising a donor monomer having the following structure (I): 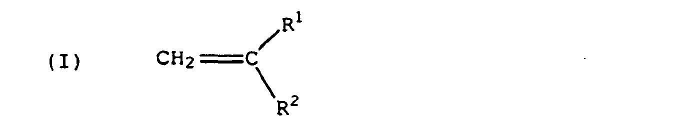 wherein R¹ is linear or branched C₁ to C₆ alkyl, R² is selected from the group consisting of linear, cyclic or branched C₁ to C₂₀ alkyl, alkenyl, C₆ to C₂₀ aryl, alkaryl and aralkyl, at least 15 mol % of the copolymer comprising an acrylic monomer as an acceptor monomer; the copolymer containing pendant carbamate groups or groups that can be converted to carbamate groups;
b) at least one aldehyde; and
c) at least one monohydric alcohol;
wherein when the copolymer (a) contains groups that can be converted to carbamate groups, the reactants further comprise:
d) at least one material that will convert said groups into carbamate groups.

2. A compound that is a copolymer comprising at least 30 mol % of residues having the following alternating structural units:
-[DM-AM]-
wherein DM represents a residue from a donor monomer, AM represents a residue from an acceptor monomer, at least 15 mol % of the copolymer comprising a donor monomer having the following structure (I): 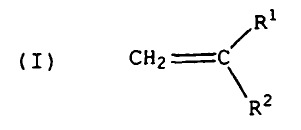 wherein R¹ is linear or branched C₁ to C₆ alkyl, R² is selected from the group consisting of linear, cyclic or branched C₁ to C₂₀ alkyl, alkenyl, C₆ to C₂₀ aryl, alkaryl and aralkyl, at least 15 mol % of the copolymer comprising an acrylic monomer as an acceptor monomer; the copolymer containing pendant groups of the structure:
-OC(O)N(R")CH₂OR'
where R' is alkyl containing one to eight carbon atoms and R" is selected from H, CH₂OR', linear, cyclic or branched C₁ to C₂₀ alkyl, alkenyl, C₆ to C₂₀ aryl, alkaryl and aralkyl.

3. The compound of any of claims 1 and 2, wherein the donor monomer is one or more selected from the group consisting of isobutylene, diisobutylene, dipentene, and isoprenol optionally in combination with styrene, substituted styrenes, methyl styrene, substituted methyl styrenes, vinyl ethers, vinyl esters, and vinyl pyridine.

4. The compound of any of claims 1 and 2, wherein the donor monomer of structure I is selected from the group consisting of isobutylene, diisobutylene, dipentene, isoprenol, and mixtures thereof.

5. The compound of claim 1, wherein the group R² of the donor monomer of structure I contains hydroxyl functionality.

6. The compound of claim 1, wherein the acceptor monomer comprises one or more described by the structure (II): 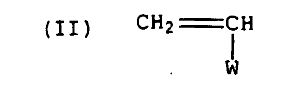 wherein W is selected from the group consisting of linear or branched C₁ to C₂₀ alkyl and alkylol.

7. The compound of claim 1, wherein the acrylic monomer is one or more described by structure (III): 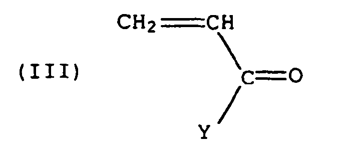 wherein Y is -OR⁴, and R⁴ is linear or branched C₁ to C₂₀ alkyl, alkylol or carbamoyl alkyl.

8. The compound of claim 7, wherein Y includes at least one hydroxyl group or carbamate group.

9. The compound of any of claims 1 and 2, wherein the copolymer has a molecular weight of from 250 to 100,000.

10. The compound of any of claims 1 and 2, wherein the copolymer has a polydispersity index of less than 4.

11. The compound of any of claims 1 and 2, wherein the alternating structural units comprise at least 50 mol % of the copolymer.

12. The compound of claim 1, wherein the acceptor monomer is one or more selected from the group consisting of hydroxyethyl acrylate, hydroxypropyl acrylate, 4-hydroxybutyl acrylate, 2-carbamoyloxyethylacrylate, and 2-carbamoyloxypropyl acrylate.

13. The compound of claim 12, wherein the acceptor monomer is 4-hydroxybutyl acrylate.

14. The compound of any of claims 1 and 2, wherein the copolymer comprises one or more residues derived from other ethylenically unsaturated monomers of general formula VII: 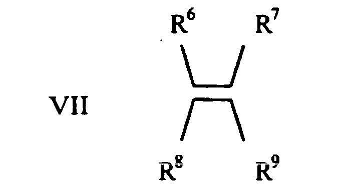 wherein R⁶, R⁷, and R⁹ are independently selected from the group consisting of H, CF₃, straight or branched alkyl of 1 to 20 carbon atoms, aryl, unsaturated straight or branched alkenyl or alkynyl of 2 to 10 carbon atoms, unsaturated straight or branched alkenyl of 2 to 6 carbon atoms substituted with a halogen, C₃-C₈ cycloalkyl, heterocyclyl and phenyl, R⁸ is selected from the group consisting of H, C₁-C₆ alkyl, and COOR¹⁰, wherein R¹⁰ is selected from the group consisting of H, an alkali metal, a C₁ to C₆ alkyl group, and C₆ to C₂₀ aryl.

15. The compound of claim 14, wherein the other ethylenically unsaturated monomers are one or more selected from the group consisting of (meth)acrylic monomers and allylic monomers.

16. The compound of claim 1 wherein the groups that can be converted to carbamate groups are hydroxyl groups and (d) is a carbamate-containing material that is reactive with the hydroxyl groups.

17. The compound of claim 16 wherein (d) is methyl carbamate.

18. The compound of claim 1 wherein the aldehyde is formaldehyde.

19. The compound of claim 1 wherein the monohydric alcohol is selected from at least one of methanol, ethanol, n-propanol, isopropanol, n-butanol, isobutanol, and cyclohexanol.

20. The compound of any of claims 1 and 2 wherein the copolymer is substantially free of maleate monomer segments and fumarate monomer segments.

21. The compound of claim 1 wherein the copolymer is prepared in the absence of Lewis acids and transition metals.

22. The compound of any of claims 1 and 2, having an equivalent weight of from 125 to 3000, based on etherified carbamate functional groups.

23. The compound of claim 2 wherein R¹ is selected from at least one of methyl, ethyl, n-propyl, isopropyl, n-butyt, isobutyl, and cyclohexyl.

24. A curable composition comprising the compound of any of claims 1 and 2.

25. A curable composition comprising:
a) the compound of any of claims 1 and 2 and
b) at least one material having functional groups that are reactive with the reaction product of a).

26. The curable composition of claim 25, wherein the compound a) is present in an amount of 1 to 99 percent by weight based on the total weight of resin solids in the curable composition.

27. The curable composition of claim 25, wherein the material b) is present in an amount of 1 to 99 percent by weight based on the total weight of resin solids in the curable composition.

28. The curable composition of claim 25, wherein the material b) has functional groups selected from the group consisting of hydroxyl, methylol, methylol ether, carboxylic acid, amide, thiol, urea, carbamate, thiocarbamate, and mixtures thereof.

29. The curable composition of claim 28, wherein the material b) is a polymer selected from the group consisting of acrylic, polyester, polyether and polyurethane polymers including mixtures thereof.

30. The curable composition of claim 25 wherein the material b) is an aminoplast.

31. The curable composition of claim 29, further comprising at least one auxiliary crosslinking agent different from a) and b), present in amounts of 1 to 50 percent by weight based on total weight of resin solids in the curable composition.

32. The curable composition of claim 31 in as much component a is the compound according to claim 1, wherein the auxiliary crosslinking agent is selected from at least one of polyisocyanates; triazine compounds of the formula: C₃N₃(NHCOXR)₃, wherein X is nitrogen, oxygen, sulfur, phosphorus, or carbon, and R is a lower alkyl group having one to twelve carbon atoms, or mixtures of lower alkyl groups; and aminoplasts; and the material b) has functional groups that are reactive with the auxiliary crosslinking agent.

33. The curable composition of claim 31 in as much component a is the compound according to claim 2, wherein the auxiliary crosslinking agent is a polyisocyanate and the polymer has functional groups that are reactive with the polyisocyanate.

34. The curable composition of any of claims 32 and 33, wherein the auxiliary crosslinking agent is a polyisocyanate, and wherein at least a portion of the isocyanate groups are capped.

35. The curable composition of claim 29 wherein the material b) is an acrylic polymer.

36. The curable composition of claim 29, wherein the material b) is present in an amount of 1 to 50 percent by weight, preferably 1 to 35 percent by weight based on the total weight of resin solids in the curable composition.

37. The curable composition of claim 35, wherein the acrylic polymer is present in an amount of 20 to 85 percent by weight based on the total weight of resin solids in the curable composition.

38. The curable composition of claim 35, wherein the acrylic polymer has hydroxyl functional groups.

39. The curable composition of claim 28, further comprising an additional polymer different from the material b), selected from acrylic polymers, polyester polymers, polyurethane polymers, and mixtures thereof.

40. The curable composition of claim 39, wherein the additional polymer is present in an amount of up to 30 percent by weight based on the total weight of resin solids in the curable composition.

41. The curable composition of claim 40, wherein in case component a is the compound according to claim 1 the additional polymer is a polyester polymer.

## Patentansprüche

1. Verbindung, die das Reaktionsprodukt von Reaktanten ist, wobei die Reaktanten umfassen:
a) wenigstens ein Copolymer, das wenigstens 30 Mol-% an Resten mit den folgenden alternierenden Struktureinheiten enthält:
-[DM-AM]-,
worin DM für einen Rest aus einem Donormonomer steht, AM für einen Rest aus einem Akzeptormonomer steht, wenigstens 15 Mol-% des Copolymers ein Donormonomer mit der folgenden Struktur umfassen (1): 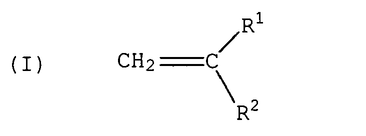 worin R¹ lineares oder verzweigtes C₁- bis C₆-Alkyl ist, R² ausgewählt ist aus der Gruppe bestehend aus linearem, cyclischem oder verzweigtem C₁- bis C₂₀-Alkyl, Alkenyl, C₆- bis C₂₀-Aryl, Alkaryl und Aralkyl, wenigstens 15 Mol-% des Copolymers ein Acrylmonomer als Akzeptormonomer umfassen und das Copolymer seitenständige Carbamatgruppen oder Gruppen enthält, die zu Carbamatgruppen umgewandelt werden können,
b) wenigstens ein Aldehyd und
c) wenigstens einen Monohydroxyalkohol,
wobei, wenn das Copolymer (a) Gruppen enthält, die zu Carbamatgruppen umgewandelt werden können, die Reaktanten zusätzlich umfassen:
d) wenigstens ein Material, das diese Gruppen zu Carbamatgruppen umwandelt.

2. Verbindung, die ein Copolymer ist, das wenigstens 30 Mol-% an Resten mit den folgenden alternierenden Struktureinheiten enthält:
-[DM-AM]-,
worin DM für einen Rest aus einem Donormonomer steht, AM für einen Rest aus einem Akzeptormonomer steht, wenigstens 15 Mol-% des Copolymers ein Donormonomer mit der folgenden Struktur (1) umfassen: 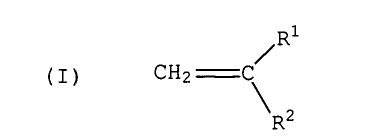 worin R¹ lineares oder verzweigtes C₁- bis C₆-Alkyl ist, R² ausgewählt ist aus der Gruppe bestehend aus linearem, cyclischem oder verzweigtem C₁-bis C₂₀-Alkyl, Alkenyl, C₆- bis C₂₀-Aryl, Alkaryl und Aralkyl, wenigstens 15 Mol-% des Copolymers ein Acrylmonomer als Akzeptormonomer umfassen, wobei das Copolymer seitenständige Gruppen der Struktur enthält:
-OC(O)N(R")CH₂OR',
worin R' Alkyl mit 1 bis 8 Kohlenstoffatomen ist und R" ausgewählt ist aus H, CH₂OR', linearem, cyclischem oder verzweigtem C₁- bis C₂₀-Alkyl, Alkenyl, C₆- bis C₂₀-Aryl, Alkaryl und Aralkyl.

3. Verbindung nach einem der Ansprüche 1 und 2, wobei das Donormonomer eins oder mehrere ist, die ausgewählt sind aus der Gruppe bestehend aus Isobutylen, Diisobutylen, Dipenten und Isoprenol, optional in Kombination mit Styrol, substituierten Styrolen, Methylstyrol, substituierten Methylstyrolen, Vinylethern, Vinylestern und Vinylpyridin.

4. Verbindung nach einem der Ansprüche 1 und 2, wobei das Donormonomer der Struktur I ausgewählt ist aus der Gruppe bestehend aus Isobutylen, Diisobutylen, Dipenten, Isoprenol und Mischungen davon.

5. Verbindung nach Anspruch 1, wobei die Gruppe R² des Donormonomers der Struktur I Hydroxylfunktionalität enthält.

6. Verbindung nach Anspruch 1, wobei das Akzeptormonomer eins oder mehrere enthält, die durch die Struktur (II) beschrieben sind: 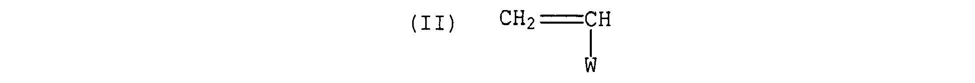 worin W ausgewählt ist aus der Gruppe bestehend aus linearem oder verzweigtem C₁- bis C₂₀-Alkyl und Alkylol.

7. Verbindung nach Anspruch 1, wobei das Acrylmonomer eins oder mehrere sind, die durch die Struktur (III) beschrieben sind: 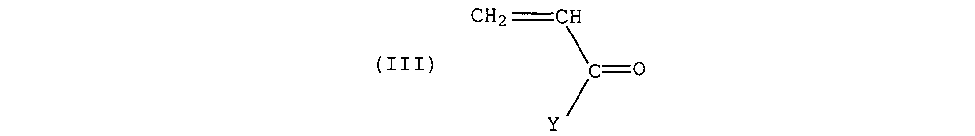 worin Y gleich -OR⁴ ist und R⁴ lineares oder verzweigtes C₁- bis C₂₀-Alkyl, Alkylol oder Carbamoylalkyl ist.

8. Verbindung nach Anspruch 7, wobei Y wenigstens eine Hydroxylgruppe oder Carbamatgruppe enthält.

9. Verbindung nach einem der Ansprüche 1 und 2, wobei das Copolymer ein Molekulargewicht von 250 bis 100.000 aufweist.

10. Verbindung nach einem der Ansprüche 1 und 2, wobei das Copolymer einen Polydispersitätsindex von weniger als 4 aufweist.

11. Verbindung nach einem der Ansprüche 1 und 2, wobei die alternierenden Struktureinheiten wenigstens 50 Mol-% des Copolymers ausmachen.

12. Verbindung nach Anspruch 1, wobei das Akzeptormonomer eins oder mehrere sind, die ausgewählt sind aus der Gruppe bestehend aus Hydroxyethylacrylat, Hydroxypropylacrylat, 4-Hydroxybutylacrylat, 2-Carbamoyloxyethylacrylat und 2-Carbamoyloxypropylacrylat.

13. Verbindung nach Anspruch 12, wobei das Akzeptormonomer 4-Hydroxybutylacrylat ist.

14. Verbindung nach einem der Ansprüche 1 und 2, wobei das Copolymer einen oder mehrere Reste enthält, die sich von anderen ethylenisch ungesättigten Monomeren der allgemeinen Formel VII ableiten: 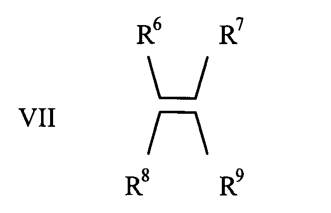 worin R⁶, R⁷ und R⁹ unabhängig voneinander ausgewählt sind aus der Gruppe bestehend aus H, CF₃, geradkettigem oder verzweigtem Alkyl mit 1 bis 20 Kohlenstoffatomen, Aryl, ungesättigtem geradkettigem oder verzweigtem Alkenyl oder Alkinyl mit 2 bis 10 Kohlenstoffatomen, ungesättigtem geradkettigem oder verzweigtem Alkenyl mit 2 bis 6 Kohlenstoffatomen, substituiert mit einem Halogen, C₃- bis C₈-Cycloalkyl, heterocyclischen Gruppen und Phenyl, R⁸ ausgewählt ist aus der Gruppe bestehend aus H, C₁- bis C₆-Alkyl und COOR¹⁰, worin R¹⁰ ausgewählt ist aus der Gruppe bestehend aus H, einem Alkalimetall, einer C₁- bis C₆-Alkylgruppe und C₆- bis C₂₀-Aryl.

15. Verbindung nach Anspruch 14, wobei die anderen ethylenisch ungesättigten Monomeren eins oder mehrere sind, ausgewählt aus der Gruppe bestehend aus (Meth)acrylmonomeren und Allylmonomeren.

16. Verbindung nach Anspruch 1, wobei die Gruppen, die zu Carbamatgruppen umgewandelt werden können, Hydroxylgruppen sind und (d) ein carbamathaltiges Material ist, das mit Hydroxylgruppen reaktiv ist.

17. Verbindung nach Anspruch 16, wobei (d) Methylcarbamat ist.

18. Verbindung nach Anspruch 1, wobei das Aldehyd Formaldehyd ist.

19. Verbindung nach Anspruch 1, wobei der Monohydroxyalkohol ausgewählt ist aus wenigstens einem von Methanol, Ethanol, n-Propanol, Isopropanol, n-Butanol, Isobutanol und Cyclohexanol.

20. Verbindung nach einem der Ansprüche 1 und 2, wobei das Copolymer im Wesentlichen frei von Maleatmonomersegmenten und Fumaratmonomersegmenten ist.

21. Verbindung nach Anspruch 1, wobei das Copolymer in Abwesenheit von Lewis-Säuren und Übergangsmetallen hergestellt ist.

22. Verbindung nach einem der Ansprüche 1 und 2 mit einem Äquivalentgewicht von 125 bis 3.000, bezogen auf die veretherten carbamatfunktionellen Gruppen.

23. Verbindung nach Anspruch 2, wobei R¹ ausgewählt ist aus wenigstens einem von Methyl, Ethyl, n-Propyl, Isopropyl, n-Butyl, Isobutyl und Cyclohexyl.

24. Härtbare Zusammensetzung, enthaltend die Verbindung nach einem der Ansprüche 1 und 2.

25. Härtbare Zusammensetzung, enthaltend:
a) die Verbindung nach einem der Ansprüche 1 und 2 und
b) wenigstens ein Material mit funktionellen Gruppen, die reaktiv mit dem Reaktionsprodukt von a) sind.

26. Härtbare Zusammensetzung nach Anspruch 25, wobei die Verbindung a) in einer Menge von 1 bis 99 Gew.-%, bezogen auf das Gesamtgewicht der Harzfeststoffe in der härtbaren Zusammensetzung, vorhanden ist.

27. Härtbare Zusammensetzung nach Anspruch 25, wobei das Material b) in einer Menge von 1 bis 99 Gew.-%, bezogen auf das Gesamtgewicht der Harzfeststoffe in der härtbaren Zusammensetzung, vorhanden ist.

28. Härtbare Zusammensetzung nach Anspruch 25, wobei das Material b) funktionelle Gruppen aufweist, die ausgewählt sind aus der Gruppe bestehend aus Hydroxyl, Methylol, Methylolether, Carbonsäure, Amid, Thiol, Harnstoff, Carbamat, Thiocarbamat und Mischungen davon.

29. Härtbare Zusammensetzung nach Anspruch 28, wobei das Material b) ein Polymer ist, ausgewählt aus der Gruppe bestehend aus Acryl-, Polyester-, Polyether- und Polyurethanpolymeren einschließlich Mischungen davon.

30. Härtbare Zusammensetzung nach Anspruch 25, wobei das Material b) ein Aminoplast ist.

31. Härtbare Zusammensetzung nach Anspruch 29, die zusätzlich wenigstens ein Hilfsvernetzungsmittel enthält, das sich von a) und b) unterscheidet und in Mengen von 1 bis 50 Gew.-%, bezogen auf das Gesamtgewicht der Harzfeststoffe in der härtbaren Zusammensetzung, vorhanden ist.

32. Härtbare Zusammensetzung nach Anspruch 31, insofern Komponente a die Verbindung gemäß Anspruch 1 ist, wobei das Hilfsvernetzungsmittel ausgewählt ist aus wenigstens einem von Polyisocyanaten, Triazinverbindungen der Formel C₃N₃(NHCOXR)₃, worin X gleich Stickstoff, Sauerstoff, Schwefel, Phosphor oder Kohlenstoff ist und R eine niedrige Alkylgruppe mit 1 bis 12 Kohlenstoffatomen oder eine Mischung von niedrigen Alkylgruppen ist, und Aminoplasten, und das Material b) funktionelle Gruppen aufweist, die mit dem Hilfsvernetzungsmittel reaktiv sind.

33. Härtbare Zusammensetzung nach Anspruch 31, insofern Komponente a die Verbindung gemäß Anspruch 2 ist, wobei das Hilfsvernetzungsmittel ein Polyisocyanat ist und das Polymer funktionelle Gruppen aufweist, die mit dem Polyisocyanat reaktiv sind.

34. Härtbare Zusammensetzung nach einem der Ansprüche 32 und 33, wobei das Hilfsvernetzungsmittel ein Polyisocyanat ist und wobei wenigstens ein Teil der Isocyanatgruppen verkappt ist.

35. Härtbare Zusammensetzung nach Anspruch 29, wobei das Material b) ein Acrylpolymer ist.

36. Härtbare Zusammensetzung nach Anspruch 29, wobei das Material b) in Mengen von 1 bis 50 Gew.-%, vorzugsweise 1 bis 35 Gew.-%, bezogen auf das Gesamtgewicht der Harzfeststoffe in der härtbaren Zusammensetzung, vorhanden ist.

37. Härtbare Zusammensetzung nach Anspruch 35, wobei das Acrylpolymer in Mengen von 20 bis 85 Gew.-%, bezogen auf das Gesamtgewicht der Harzfeststoffe in der härtbaren Zusammensetzung, vorhanden ist.

38. Härtbare Zusammensetzung nach Anspruch 35, wobei das Acrylpolymer hydroxyfunktionelle Gruppen aufweist.

39. Härtbare Zusammensetzung nach Anspruch 28, die ferner ein zusätzliches Polymer enthält, das sich von dem Material b) unterscheidet, ausgewählt aus Acrylpolymeren, Polyesterpolymeren, Polyurethanpolymeren und Mischungen davon.

40. Härtbare Zusammensetzung nach Anspruch 39, wobei das zusätzliche Polymer in Mengen von bis zu 30 Gew.-%, bezogen auf das Gesamtgewicht der Harzfeststoffe in der härtbaren Zusammensetzung, vorhanden ist.

41. Härtbare Zusammensetzung nach Anspruch 40, wobei für den Fall, dass Komponente a die Verbindung gemäß Anspruch 1 ist, das zusätzliche Polymer ein Polyesterpolymer ist.

## Revendications

1. Composé qui est le produit de réaction de réactifs, où les réactifs comprennent :
a) au moins un copolymère comprenant au moins 30% molaires de résidus possédant les unités structurelles alternées suivantes :
-[DM - AM]
où DM représente un résidu d'un monomère donneur, AM représente un résidu d'un monomère accepteur, au moins 15% molaires du copolymère comprenant un monomère donneur ayant la structure suivante : 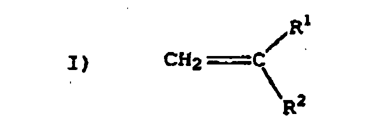 dans laquelle R¹ est un radical alkyle en C₁ à C₆ linéaire ou ramifié, R² est choisi dans le groupe formé par des radicaux alkyle, alcényle en C₁ à C₂₀ linéaires, cycliques ou ramifiés, aryle, alkaryle et aralkyle en C₆ à C₂₀, au moins 15% molaires du copolymère comprenant un monomère acrylique en tant que monomère accepteur, le copolymère contenant des groupes carbamate pendants ou des groupes pouvant être convertis en groupes carbamate,
b) au moins un aldéhyde, et
c) au moins un alcool monovalent,
où, lorsque le copolymère (a) contient des groupes qui peuvent être convertis en groupes carbamate, les réactifs comprennent en outre :
d) au moins une matière qui convertira lesdits groupes en groupes carbamate.

2. Composé qui est un copolymère comprenant au moins 30% molaires de résidus possédant les unités structurelles alternées suivantes :
-[DM - AM]
où DM représente un résidu d'un monomère donneur, AM représente un résidu d'un monomère accepteur, au moins 15% molaires du copolymère comprenant un monomère donneur ayant la structure suivante : 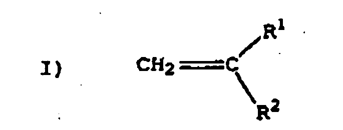 dans laquelle R¹ est un radical alkyle en C₁ à C₆ linéaire ou ramifié, R² est choisi dans le groupe formé par des radicaux alkyle, alcényle en C₁ à C₂₀ linéaires, cycliques ou ramifiés, aryle, alkaryle et aralkyle en C₆ à C₂₀, , au moins 15% molaires du copolymère comprenant un monomère acrylique en tant que monomère accepteur, le copolymère contenant des groupes pendants de la structure suivante :
-OC(O)N(R")CH₂OR
dans laquelle R' est un radical alkyle comportant de un à huit atomes de carbone et R" est choisi parmi H, CH₂OR' alkyle, alcényle en C₁ à C₂₀ linéaire, cyclique ou ramifié, aryle, alkaryle et aralkyle en C₆ à C₂₀.

3. Composé suivant l'une quelconque des revendications 1 et 2, dans lequel le monomère donneur est un ou plusieurs composés choisis dans le groupe formé par l'isobutylène, le diisobutylène, le dipentène et l'isoprénol, éventuellement en combinaison avec du styrène, des styrènes substitués, du méthylstyrène, des méthylstyrènes substitués, des éthers vinyliques, des esters vinyliques et de la vinylpyridine.

4. Composé suivant l'une quelconque des revendications 1 et 2, dans lequel le monomère donneur de la structure I est choisi dans le groupe formé par l'isobutylène, le diisobutylène, le dipentène, l'isoprénol et leurs mélanges.

5. Composé suivant la revendication 1, dans lequel le groupe R² du monomère donneur de la structure I contient une fonctionnalité hydroxyle.

6. Composé suivant la revendication 1, dans lequel le monomère accepteur comprend un ou plusieurs composés décrits par la structure (II) : 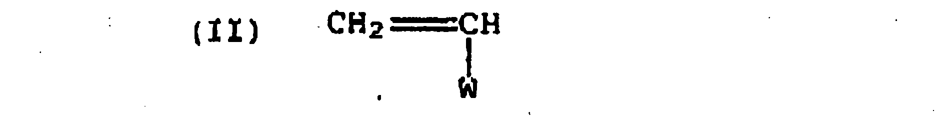 dans laquelle W est choisi dans le groupe formé par un alkylol et un alkyle linéaire ou ramifié en C₁ à C₂₀.

7. Composé suivant la revendication 1, dans lequel le monomère acrylique est un ou plusieurs monomères décrits par la structure (III) : 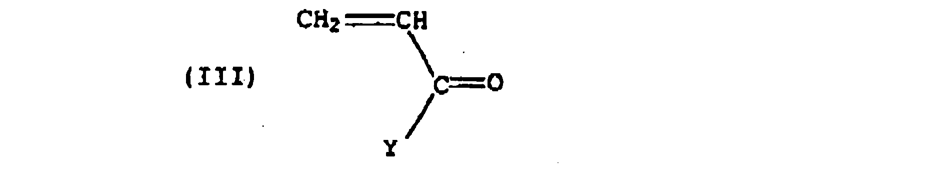 dans laquelle Y représente -OR⁴, et R⁴ est un alkyle, alkylol ou carbamoylalkyle linéaire ou ramifié en C₁ à C₂₀

8. Composé suivant la revendication 7, dans lequel Y comprend au moins un groupe hydroxyle ou un groupe carbamate.

9. Composé suivant l'une quelconque des revendications 1 et 2, dans lequel le copolymère a un poids moléculaire de 250 à 100 000.

10. Composé suivant l'une quelconque des revendications 1 et 2, dans lequel le copolymère a un indice de polydispersivité de moins de 4.

11. Composé suivant l'une quelconque des revendications 1 et 2, dans lequel les unités structurelles alternatives comprennent au moins 50% molaires du copolymère.

12. Composé suivant la revendication 1, dans lequel le monomère accepteur est un ou plusieurs monomères choisis dans le groupe formé par l'acrylate d'hydroxyéthyle, l'acrylate d'hydroxypropyle, l'acrylate de 4-hydroxybutyle, l'acrylate de 2-carbamoyloxyéthyle et l'acrylate de 2-carbamoyloxypropyle.

13. Composé suivant la revendication 12, dans lequel le monomère accepteur est de l'acrylate de 4-hydroxybutyle.

14. Composé suivant l'une quelconque des revendications 1 et 2, dans lequel le copolymère comprend un ou plusieurs résidus dérivés d'autres monomères éthyléniquement insaturés de la formule générale VII : 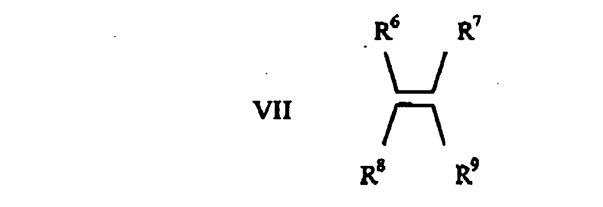 dans laquelle R⁶, R⁷et R⁹ sont choisis indépendamment dans le groupe formé par H, CH₃, alkyle linéaire ou ramifié comportant de 1 à 20 atomes de carbone, aryle, alcényle ou alcynyle insaturés, linéaires ou ramifiés comportant de 2 à 10 atomes de carbone, alcényle insaturé, linéaire ou ramifié comportant de 2 à 6 atomes de carbone, substitué par un halogène, cycloalkyle en C₃ à C₈, hétérocyclyle et phényle, R⁸ est choisi dans le groupe formé par H, alkyle en C₁ à C₆, et COOR¹⁰, où R¹⁰ est choisi dans le groupe formé par H, un métal alcalin, un groupe alkyle en C₁ à C₆ et aryle en C₆ à C₂₀.

15. Composé suivant la revendication 14, dans lequel les autres monomères éthyléniquement insaturés sont un ou plusieurs monomères choisis dans le groupe formé par des monomères (méth)acryliques et des monomères allyliques.

16. Composé suivant la revendication 1, dans lequel les groupes qui peuvent être convertis en groupes carbamates sont des groupes hydroxyle et (d) est une matière contenant du carbamate qui est réactive vis-à-vis des groupes hydroxyle.

17. Composé suivant la revendication 16, dans lequel (d) est du carbamate de méthyle.

18. Composé suivant la revendication 1, dans lequel l'aldéhyde est du formaldéhyde.

19. Composé suivant la revendication 1, dans lequel l'alcool monovalent est choisi parmi au moins un des méthanol, éthanol, n-propanol, isopropanol, n-butanol, isobutanol et cyclohexanol.

20. Composé suivant l'une quelconque des revendications 1 et 2, dans lequel le copolymère est substantiellement exempt de segments monomères de maléate et de segments monomères de fumarate.

21. Composé suivant la revendication 1, dans lequel le copolymère est préparé en l'absence d'acides de Lewis et de métaux de transition.

22. Composé suivant l'une quelconque des revendications 1 et 2, ayant un poids équivalent de 125 à 3000, sur la base des groupes fonctionnels carbamate éthérifiés.

23. Composé suivant la revendication 2, dans lequel R¹ est choisi parmi au moins l'un des radicaux méthyle, éthyle, n-propyle, isopropyle, n-butyle, isobutyle et cyclohexyle.

24. Composition durcissable comprenant le composé suivant l'une quelconque des revendications 1 et 2.

25. Composition durcissable comprenant :
a) le composé suivant l'une quelconque des revendications 1 ou 2,
b) au moins une matière possédant des groupes fonctionnels réactifs avec le produit de réaction de a).

26. Composition durcissable suivant la revendication 25, dans laquelle le composé a) est présent en une quantité de 1 à 99% en poids, sur la base du poids total de solides de la résine dans la composition durcissable.

27. Composition durcissable suivant la revendication 25, dans laquelle la matière b) est présente en une quantité de 1 à 99% en poids, sur la base du poids total de solides de la résine dans la composition durcissable.

28. Composition durcissable suivant la revendication 25, dans laquelle la matière b) possède des groupes fonctionnels choisis dans le groupe formé par des groupes hydroxyle, méthylol, méthyloléther, acide carboxylique, amide, thiol, urée, carbamate, thiocarbamate et leurs mélanges.

29. Composition durcissable suivant la revendication 28, dans laquelle la matière b) est un polymère choisi dans le groupe formé par des polymères acryliques, des polyesters, des polyéthers et des polyuréthannes, ceci comprenant leurs mélanges.

30. Composition durcissable suivant la revendication 25,
dans laquelle la matière b) est un aminoplaste.

31. Composition durcissable suivant la revendication 29, comprenant en outre au moins un agent réticulant auxiliaire différent de a) et b), présent en des quantités de 1 à 50% en poids, sur la base du poids total de solides de la résine dans la composition durcissable.

32. Composition durcissable suivant la revendication 31, dans laquelle le composant a est le composé suivant la revendication 1, où l'agent réticulant auxiliaire est choisi parmi au moins un des suivants : des polyisocyanates, des composés de triazine de la formule C₃N₃(NHCOXR)₃, où X est de l'azote, de l'oxygène, du soufre, du phosphore ou du carbone, et R est un groupe alkyle inférieur comportant de un à douze atomes de carbone, ou des mélanges de groupes alkyle inférieurs, et des aminoplastes, et la matière b) possède des groupes fonctionnels réactifs avec l'agent réticulant auxiliaire.

33. Composition durcissable suivant la revendication 31, dans laquelle le composant a est le composé suivant la revendication 2, où l'agent réticulant auxiliaire est un polyisocyanate et le polymère possède des groupes fonctionnels réactifs avec le polyisocyanate.

34. Composition durcissable suivant l'une quelconque des revendications 32 et 33, dans laquelle l'agent réticulant auxiliaire est un polyisocyanate, et où au moins une partie des groupes isocyanate sont bloqués.

35. Composition durcissable suivant la revendication 29, dans laquelle la matière b) est un polymère acrylique.

36. Composition durcissable suivant la revendication 29, dans laquelle la matière b) est présente en une quantité de 1 à 50% en poids, de préférence de 1 à 35% en poids, sur base du poids total de solides de la résine dans la composition durcissable.

37. Composition durcissable suivant la revendication 35, dans laquelle le polymère acrylique est présent en une quantité de 20 à 85% en poids, sur base du poids total de solides de la résine dans la composition durcissable.

38. Composition durcissable suivant la revendication 35, dans laquelle le polymère acrylique possède des groupes fonctionnels hydroxyle.

39. Composition durcissable suivant la revendication 28, comprenant en outre un polymère additionnel différent de la matière b), choisi parmi des polymères acryliques, des polymères polyesters, des polymères polyuréthannes et leurs mélanges.

40. Composition durcissable suivant la revendication 39, dans laquelle le polymère additionnel est présent en une quantité allant jusqu'à 30% en poids, sur base du poids total de solides de la résine dans la composition durcissable.

41. Composition durcissable suivant la revendication 40, dans laquelle, dans le cas où le composant a est le composé suivant la revendication 1, le polymère additionnel est un polymère polyester.
